# EUROPEAN PATENT APPLICATION

(11) **EP 4 560 316 A1**
(43) Date of publication of application: **28.05.2025**
(21) Application number: 23383205.4
(22) Date of filing: 23.11.2023
(51) Int. Cl.: G01N 33/569

(54) **ANTIGENIC REGIONS FOR THE DETECTION OF ANTI-TOXOPLASMA GONDII IGM ANTIBODIES**

(71) Applicant: Biokit Research & Development S.L.U., 08186 Lliçà d'Amunt Barcelona (ES); Universitat Pompeu Fabra, 08002 Barcelona (ES)
(72) Inventor: LLEVADOT ESQUERDA, Roser, 08186 Barcelona (ES); PALICIO HERRERO, Marta, 08186 Barcelona (ES); RIPOLL PASTOR, Greta, 08186 Barcelona (ES); ANDREU MARTÍNEZ, David, 08003 Barcelona (ES)
(74) Representative: Hoffmann Eitle

(57) **Abstract**

The present invention provides chimeric, antigenic peptides which are suitable for the detection and diagnosis of *Toxoplasma gondii* infections.

## Description

### Technical field

The present invention provides compounds, methods for their preparation, methods for their use and compositions containing them, which are suitable for industrial application in the pharmaceutical and diagnostic/prognostic field, particularly for the detection and diagnosis of *Toxoplasma gondii* infections, as well as for the treatment and prevention of said infections.

### Background of the invention

The diagnosis of acute toxoplasmosis is based mainly on serological tests that detect anti-T. *gondii* IgM antibodies. The commercial kits available in the market are usually based on *T. gondii* lysate antigens (TLA) from different sources (i.e., infected mice and/or tissue cultures). The use of *T. gondii* lysate antigens present inherent limitations in terms of performance (sensitivity and specificity, difficult standardization and strong regulation. In recent years, purified recombinant antigens have become a promising alternative with proved diagnostic utility. However, several problems are associated with their use for the serological diagnosis of *T. gondii* infection such as low inter-assay reproducibility, laborious and expensive production and low immunoreactivity. As a result, none of the assays based on recombinant antigens have all the characteristics required to replace the *T. gondii* lysate antigens to detect IgM. Therefore, further work is needed to improve the diagnosis/prognosis of toxoplasmosis, such improvement shall have a direct and positive impact on the clinical management of infected patients. In this sense, to achieve such improvement, discovering new epitopes that could help in the clinical management of toxoplasmosis is one of the main foci of this invention.

### Description

Serologic tests for detecting anti-*T*. *gondii* IgMs are the gold standard for the diagnosis of acute toxoplasmosis. Nevertheless, most commercial kits are based on lysate antigens obtained from tachyzoites grown in mice or tissue culture which present inherent limitations in terms of performance, providing erroneous results to the physicians, thus hindering patient management and requiring stringent regulation. In this context, peptide-based antigens have emerged as an attractive alternative to overcome several of such issues. In this sense, in the present invention we focus on designing and synthetizing *T. gondii* IgM-specific chimeric peptides to develop an immunoassay for the specific detection of anti-*T*. *gondii* IgMs. Additionally, we aimed to assess mouse monoclonal humanized chimeric antibodies as an alternative source to the human positive plasma-derived samples that are currently used to produce reference materials, such as calibrators and controls.

More particularly and focusing on the *T. gondii* IgM-specific chimeric peptide that was used to develop an immunoassay for the detection of anti-*T*. *gondii* IgMs. In the present invention, to identify new *T. gondii* epitopes that could be incorporated as diagnostic tools in an immunoassay for specifically detecting anti-*T*. *gondii* IgM, we designed a peptide microarray that displayed all peptides covering different antigenic proteins from *T*. *gondii.* A complete list of the nine proteins selected, identified by means of UniProt (https://www.unipro*T*.org/) and the Immune Epitope Database (IEDB) (https://www.iedb.org/), is shown in table 1 below:

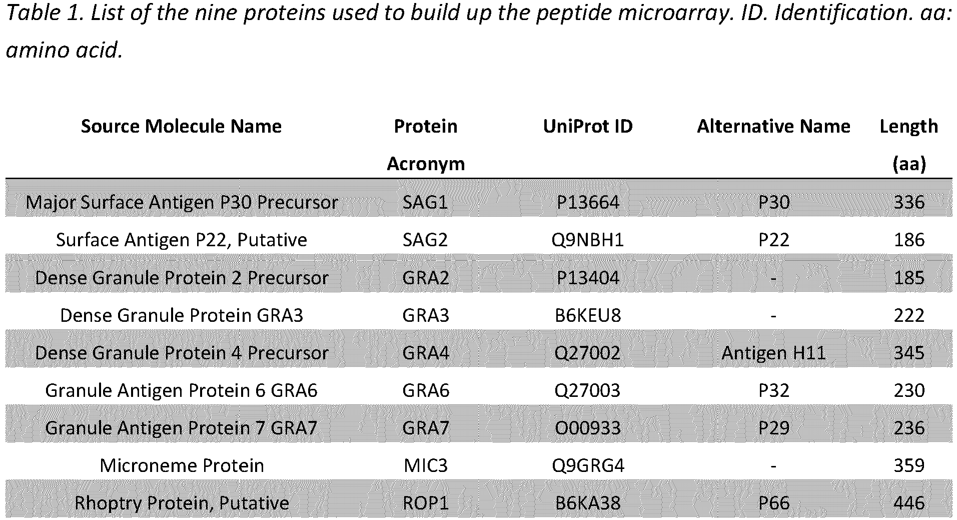

From the information in the differential IgM response heatmap, provided in the examples of the present invention, top peptide hits exhibited G1-G2 differences of >400 units, which were predominantly assigned to dense granule protein GRA3, rhoptry protein, granule antigen protein GRA7 and major surface antigen P30.

Based on the above results of the microarray analysis, we identified the sequences that had the potential to perform best. We based the selection on the following:
i) IgM specificity based on G1-G2 differences and G1/G2 ratio for differential IgM responses,
ii) homology to *T. gondii* B cell epitopes found in IEDB, and
iii) peptide length.

Thus, we designed the peptides to include the areas within the studied proteins that were most specific for IgM but limiting peptide length to around 50-60 residues, which is the approximate threshold after which the quality of synthetically produced peptides could be compromised. The 18 selected peptides, were synthesized in C-terminal carboxamide form using Fmoc solid phase synthesis, purified to >95% homogeneity using HPLC (high performance liquid chromatography) and satisfactorily characterized using MS (mass spectrometry). (see Table 2 below).

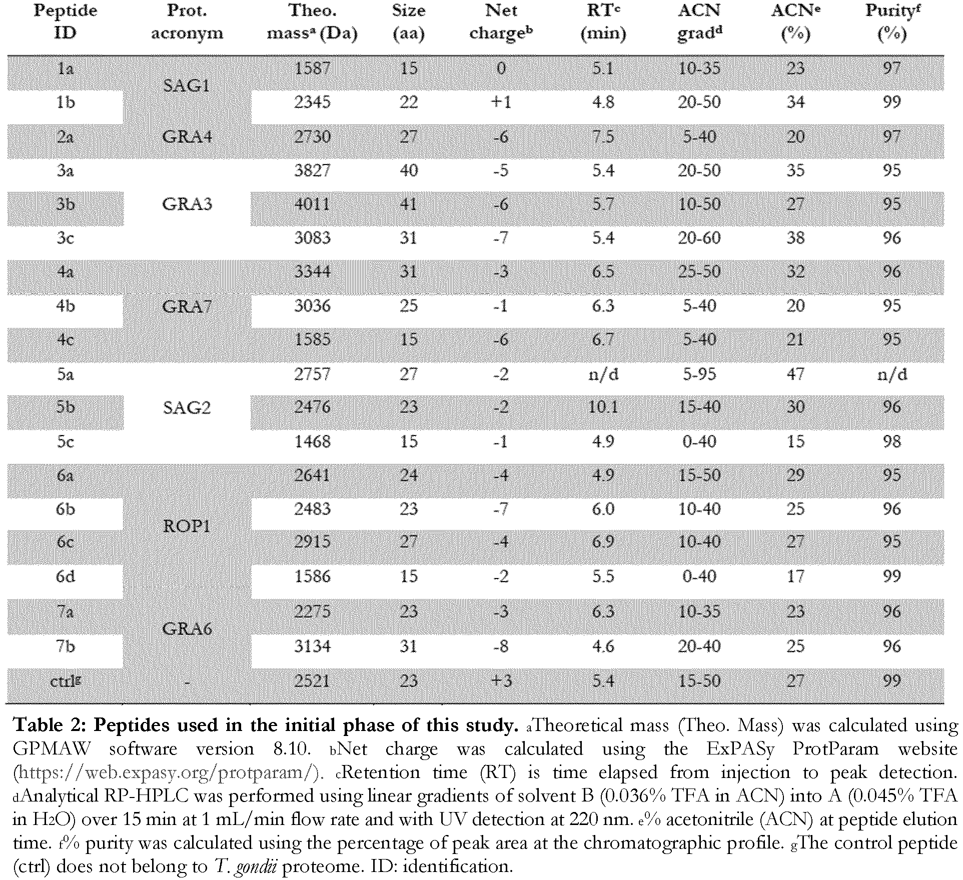

The microarray study thus elucidated 18 different peptides that potentially reacted with IgM anti*-T*. *gondii* positive samples and did not react with anti-*T*. *gondii* IgG-positive samples. In addition, the results obtained with a multiplex ELISA indicated that the best candidates **were peptides 3a, 3b** (GRA3) **and 6c** (ROP1).

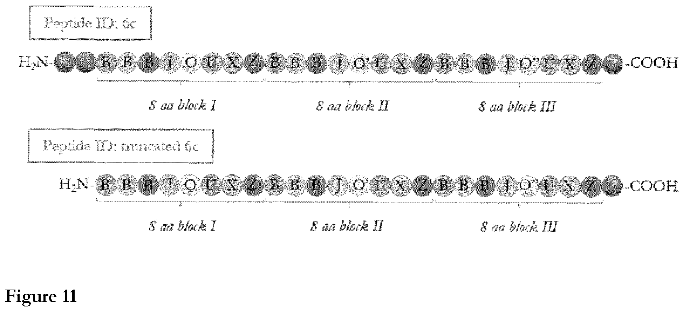

Next, we evaluated the diagnostic utility of different chimeric peptides based on two antigenic regions of GRA3 and ROP1 . Considering that, we demonstrated that the combination in solution of 3a and 6c peptides enhanced assay performance compared with the peptides alone, we designed and produced different synthetic chimeric peptides combining such peptides and evaluated their performance in a singleplex ELISA. Overall, the results demonstrated that synthetic chimeric peptide 3a6c can be used in a chemiluminescent immunoassay replacing *T. gondii* parasite native proteins and being especially useful to detect IgMs anti-*T*. *gondii* in human sera. It is worth mentioning that an interesting feature of peptide 6c is that it is composed of a tandem repeat of eight residues (PPPNXQEL, wherein X can be any of aminoacids S or A). Given the presence of those block-tandem sequences in the ROP1 protein we herein show that these eight residues are involved in recognizing IgM. On that basis, we designed and produced other chimeric peptides with more than one repetition of the 6c sequence minus the two N-terminal residues (hereinafter named truncated 6c*) as a motif for three different constructions, namely i) homotandem with two consecutive stretches of truncated peptide 6c (ID 6c*2), ii) homotandem with two truncated 6c peptide sequences separated by a flexible spacer (8-amino-3,6-dioxaoctanoic acid (O2Oc)) (ID 6cO₃6c^{†}), and iii) branched bivalent peptide based on a lysine core from which two truncated 6c peptide sequences branched out (ID (6c*)2K3) (Figure 11). The results show, as illustrated in table 20, that all of these antigens provide an AUC greater than 0.80 and are thus useful for detecting anti-*T*. *gondii* IgMs and for the diagnosis of acute toxoplasmosis (see table 17) .

Therefore, a first aspect of the invention refers to an antigen, preferably a recombinant or synthetic antigen, comprising one or more amino acid sequences PPPNXQEL, wherein X can be any of amino acids S or A, and wherein the antigen is capable of specifically binding to *Toxoplasma* gondii-specific IgM antibodies. Preferably the *Toxoplasma* gondii-specific antibodies are extracted from sera of subjects who have been infected by *Toxoplasma gondii.*

In the context of the present invention, by "capable of specifically binding to" mean that, in a mixture of antibodies of multiple classes, the antigen, preferably a recombinant or synthetic antigen, of the present invention preferentially binds to antibodies of the IgM class wherein at least about 50% of the peptide binds IgM antibodies, more preferably about 70%, more preferably about 80 to 85%, even more preferably about 90% and most preferably at about 95% or more of the peptide binds IgM antibodies relative to its binding to antibodies of other classes such as IgG antibodies. The terms "selective binding" and "selectively bind" mean that, in a mixture of antibodies of multiple classes as well as other serum or cellular proteins and products, the antigen, preferably a recombinant or synthetic antigen, of the present invention preferentially binds to antibodies of the IgM class wherein at least about 50% of the antigen binds IgM antibodies, more preferably about 70%, more preferably about 80 to 85%, even more preferably about 90% and most preferably at about 95% or more of the peptide binds IgM antibodies relative to its binding to antibodies of other classes.

In the context of the present invention, the term "linker or spacer" is understood as any molecule that can be added to separate different protein domains, either in a lineal or branched structure. Non-limiting spacers are amino acids, peptides, polyethylene glycol, etc.

As used herein, *"Toxoplasma gondii (T. gondii)"* is understood as an intracellular parasitic protozoan that infects most species of warm-blooded animals and causes the disease toxoplasmosis. *T. gondii* can only go through the entire cycle of reproduction in domestic and wild cats which are the main hosts for the parasite.

As used herein, "toxoplasmosis" is understood as the parasitic disease caused by *T. gondii.* Humans can become infected by any of several routes: eating undercooked meat of animals harbouring tissue cysts; consuming food or water contaminated with cat faces or by contaminated environmental samples (such as faecal-contaminated soil or changing the litter box of a pet cat); by blood transfusion or organ transplantation; or transplacentally from mother to foetus.

As used herein, "acute toxoplasmosis" is understood as the body's response to an initial infection of *T. gondii.* Acquired infection with *Toxoplasma* in immunocompetent persons is generally an asymptomatic infection. However, 10% to 20% of patients with acute infection may develop cervical lymphadenopathy and/or a flu-like illness. Congenital toxoplasmosis results from an acute primary infection acquired by the mother during pregnancy.

As used herein, "chimeric peptide" is understood as a peptide that has been created through the joining of two or more protein domains originally coded for separate proteins. The different domains can be separated by a linker or spacer.

More particularly the present invention covers any antigen or poly(amino acid) sequence comprising one, two or more of amino acid sequences consisting of PPPNXQEL, preferably three or more, more preferably four or more, capable of specifically binding to *Toxoplasma* gondii-specific IgM antibodies. It is noted that one or more tandem sequences of PPPNXQEL present in an antigen or poly(amino acid) sequence can be optionally separated within the antigen or poly(amino acid) sequence by a linker sequence. More particularly the present invention covers any antigen or poly(amino acid) sequence comprising, consisting essentially of or consisting of one, two or more of amino acid sequences consisting of PPPNXQEL or those identified in table 17, in particular any antigen or poly(amino acid) sequence comprising, consisting essentially of or consisting of any of the following sequences : SEQ ID NO 1 (antigen 6c: EVPPPNAQELPPPNSQELPPPNSQELP), SEQ ID NO 2 (6c*: PPPNAQELPPPNSQELPPPNSQELP), SEQ ID NO 3 (6c*2: PPPNAQELPPPNSQELPPPNSQELPPPNAQELPPPNSQELPPPNSQELP), and/or SEQ ID NO 4 (6cO₃6c: PPPNAQELPPPNSQELPPPNSQELOOOPPPNAQELPPPNSQELPPPNSQELP, wherein OOO is the flexible spacer 8-amino-3,6-dioxaoctanoic acid), and wherein these sequences are capable of specifically binding to *Toxoplasma* gondii-specific IgM antibodies. It is noted that the antigen or poly(amino acid) sequence of the invention can be a peptide or a protein, wherein if the antigen is a peptide the peptide shall preferably have a length of between 8 and 150 amino acids (including the upper and lower limits of this range), preferably a maximum length of 75, 100 or 150 amino acids, and if the antigen is a protein then the antigen shall preferably have a length of between 75 and 700 amino acids, preferably between 100 and 700 amino acids, more preferably between 150 and 700 amino acids (including the upper and lower limits of this range), preferably a maximum length of 700 amino acids. Preferably, the present invention covers any antigen or poly(amino acid) sequence comprising or consisting of any of the following sequences : SEQ ID NO 1, SEQ ID NO 2, SEQ ID NO 3 and/or SEQ ID NO 4, capable of specifically binding to *Toxoplasma* gondii-specific IgM antibodies, wherein the antigen can be a peptide or protein as specified above; or any antigen, or poly(amino acid) sequence, having at least 80%, 85%, 90%, 95%, 96%, 97%, 98% or at least 99% sequence identity with any one of sequences SEQ ID NO 1, SEQ ID NO 2, SEQ ID NO 3 and/or SEQ ID NO 4, capable of specifically binding to *Toxoplasma* gondii-specific IgM antibodies.

For example, the antigen or poly(amino acid) sequence of the invention, according to the first aspect, preferably comprises or consists of amino acid sequence SEQ ID NO: 1 or amino acid sequence SEQ ID NO: 2 or amino acid sequence SEQ ID NO: 3, or amino acid sequence SEQ ID NO: 4.

A second aspect of the invention refers to an antigen, preferably a recombinant antigen, or a poly(amino acid) sequence comprising or consisting of SEQ ID NO 5 (3a: FLVAAALGGLAADQPENHQALAEPVTGVGEAGVSPVNEAG) and/or SEQ ID NO 6 (3b: AADQPENHQALAEPVTGVGEAGVSPVNEAGESYSSATSGVQ), capable of specifically binding to *Toxoplasma* gondii-specific IgM antibodies; or any antigen or poly(amino acid) sequence having at least 80%, 85%, 90%, 95%, 96%, 97%, 98% or at least 99% sequence identity with any one of sequences SEQ ID NO 5 and/or SEQ ID NO 6, capable of specifically binding to *Toxoplasma* gondii-specific IgM antibodies. Preferably the *Toxoplasma* gondii-specific antibodies are extracted from sera of subjects who have been infected by *Toxoplasma gondii.* Also in the context of the second aspect of the invention, it is noted that the antigen of the invention can be a peptide or a protein, wherein if the antigen is a peptide the peptide shall preferably have a length of between 8 and 150 amino acids (including the upper and lower limits of this range), preferably a maximum length of 75, 100 or 150 amino acids, and if the antigen is a protein then the antigen shall preferably have a length of between 75 and 700 amino acids, preferably between 100 and 700 amino acids, more preferably between 150 and 700 amino acids (including the upper and lower limits of this range), preferably a maximum length of 700 amino acids.

A third aspect of the invention refers to an antigen, in particular a chimeric antigen, preferably a chimeric recombinant antigen, or a poly(amino acid) sequence comprising at least two different antigenic regions, one of these regions being a sequence selected from the first aspect of the invention and the other sequence selected from the second aspect of the invention, wherein optionally these sequences are separated by a linker. Preferably, such antigen or poly(amino acid) sequence comprises or consists of an amino acid sequence selected from the group consisting of: SEQ ID NO: 7 (3a6c) and/or SEQ ID NO 8 (6c3a), capable of specifically binding to *Toxoplasma* gondii-specific IgM antibodies; or any antigen or poly(amino acid) sequence having at least 80%, 85%, 90%, 95%, 96%, 97%, 98% or at least 99% sequence identity with any one of sequences SEQ ID NO 7 and/or SEQ ID NO 8, capable of specifically binding to *Toxoplasma* gondii-specific IgM antibodies. Also in the context of the third aspect of the invention, it is noted that the antigen of the invention can be a peptide or a protein, wherein if the antigen is a peptide the peptide shall preferably have a length of between 8 and 150 amino acids (including the upper and lower limits of this range), preferably a maximum length of 75, 100 or 150 amino acids, and if the antigen is a protein then the antigen shall preferably have a length of between 75 and 700 amino acids, preferably between 100 and 700 amino acids, more preferably between 150 and 700 amino acids (including the upper and lower limits of this range), preferably a maximum length of 700 amino acids.

The chimeric antigens of the third aspect of the invention or the tandem sequences of the first aspect of the invention may be engineered using known methods. The fusions may be direct (the C-terminus of one amino acid sequence is linked to the N-terminal of the other through a simple covalent bond) or they may employ a flexible linker domain, or polypeptide linkers consisting of small amino acids such as glycine, serine, threonine or alanine, at various lengths and combinations. For example, the linker may be a poly-glycine repeat interrupted by serine or threonine at a certain interval.

Additionally, the chimeric antigens or poly(amino acid) sequences of the third aspect of the invention, the antigens or poly(amino acid) sequences as defined in the first aspect of the invention or the antigens or poly(amino acid) sequences as defined in the second aspect of the invention may be tagged by His-His-His-His-His-His (His6), to allow rapid purification by metal-chelate chromatography, and/or by epitopes to which antibodies are available, to allow for detection on western blots, immunoprecipitation, or activity depletion/blocking in bioassays. Other tags are possible (e.g. SUMO, Strep, etc..)

In the context of the present invention, the term "antigens of the present invention" or "recombinant or synthetic antigens of the present invention" refers to any one or more of the chimeric antigens of the third aspect of the invention, the antigens as defined in the first aspect of the invention and/or the antigens as defined in the second aspect of the invention, or any combinations thereof. It is further noted that the term antigen and poly(amino acid) sequence as used herein are interchangeable.

Another object of the present invention is a nucleotide sequence coding for the chimeric antigens of the third aspect of the invention, the antigens comprising the tandem sequences of the first aspect of the invention, or the antigens as defined in the second aspect of the invention. Also included in the scope of the present invention are nucleotide sequences that hybridizes with any sequence described above under stringent hybridization conditions, as well the corresponding chimeric recombinant antigen encoded by such hybridized nucleotide sequence.

The antigens of the present invention may be prepared by cloning and expression in a prokaryotic or eukaryotic expression system, using the appropriate expression vectors. Any method known in the art can be employed. For example, the DNA molecules coding for the antigens of the invention are inserted into appropriately constructed expression vectors by techniques well known in the art (see Sambrook et al, 1989). Such vectors are another object of the present invention.

In order to be capable of expressing the desired protein (in this case the antigens), an expression vector should comprise also specific nucleotide sequences containing transcriptional and translational regulatory information linked to the DNA coding the desired protein in such a way as to permit gene expression and production of the protein. First in order for the gene to be transcribed, it must be preceded by a promoter recognizable by RNA polymerase, to which the polymerase binds and thus initiates the transcription process. There are a variety of such promoters in use, which work with different efficiencies (strong and weak promoters).

For eukaryotic hosts, different transcriptional and translational regulatory sequences may be employed, depending on the nature of the host. They may be derived from viral sources, such as adenovirus, bovine papilloma virus, Simian virus or the like, where the regulatory signals are associated with a particular gene, which has a high level of expression. Examples are the TK promoter of the Herpes virus, the SV40 early promoter, the yeast gal4 gene promoter, etc. Transcriptional initiation regulatory signals may be selected which allow for repression and activation, so that expression of the genes can be modulated. All these hosts are a further object of the present invention.

Nucleic acid molecules which encode the antigens of the invention may be ligated to a heterologous sequence so that the combined nucleic acid molecule encodes a fusion protein.

Such combined nucleic acid molecules are included within the embodiments of the invention. For example, they may be joined to the DNA coding for a protein which allows purification of the chimeric antigen by only one step of affinity chromatography. This joined/fused protein may be for example Glutathione Sulpho Transferase (GST) to generate fusion products at the carboxy terminus of GST protein. The corresponding recombinant proteins expressed in the cytoplasm of transformed E. coli cells may be purified by affinity chromatography using a Glutathione-Sepharose resin. Other fusion proteins are also considered, not only to simplify purification but also to solubilize the chimeric antigen, as for example, Thioredoxin (trx) or Maltose-binding protein (MBP).

The DNA molecule comprising the nucleotide sequence coding for any of the antigens of the present invention can be inserted into vector(s), having the operably linked transcriptional and translational regulatory signals, which is capable of integrating the desired gene sequences into the host cell. The cells which have been stably transformed by the introduced DNA can be selected by also introducing one or more markers which allow for selection of host cells which contain the expression vector. The marker may also provide for phototrophy to an auxotropic host, biocide resistance, e,g. antibiotics, or heavy metals such as copper, or the like. The selectable marker gene can either be directly linked to the DNA gene sequences to be expressed or introduced into the same cell by co-transfection. Additional elements may also be needed for the optimal synthesis of antigens of the present invention.

Once the vector(s) or DNA sequence containing the construct(s) has been prepared for expression the DNA construct(s) may be introduced into an appropriate host cell by any of a variety of suitable means: transformation, transfection, conjugation, protoplast fusion, electroporation, calcium phosphate-precipitation, direct microinjection, etc.

Host cells may be either prokaryotic or eukaryotic. Example of eukaryotic hosts are mammalian cells, such as human, monkey, mouse, and Chinese hamster ovary (CHO) cells. Expression in these host cells provides post-translational modifications to protein molecules, including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e., pre-peptides). Example of prokaryotic hosts are bacteria, such as *Escherichia coli.*

After the introduction of the vector(s), the host cells are grown in a selective medium, which selects for the growth of vector-containing cells. Expression of the cloned gene sequence(s) results in the production of the desired proteins.

Purification of the recombinant antigens is carried out by any one of the methods known for this purpose, i.e. any conventional procedure involving extraction, precipitation, chromatography, electrophoresis, or the like. A further purification procedure that may be used in preference for purifying the antigens of the invention is affinity chromatography using monoclonal antibodies which bind the target protein and which are produced and immobilized on a gel matrix contained within a column. Impure preparations containing the recombinant protein are passed through the column. The antigens will be bound to the column by the specific antibody while the impurities will pass through. After washing, the antigen is eluted from the gel by a change in pH or ionic strength.

Another aspect of the present invention is the process for the recombinant production of the antigens of the invention as described above, comprising culturing the host cell transformed with the vector containing the nucleotide sequence of the invention and isolating the desired product.

A further object of the present invention is a DNA molecule comprising the DNA sequence coding for the above fusion protein, as well as nucleotide sequences substantially the same.

Nucleotide sequences substantially the same includes all other nucleic acid sequences which, by virtue of the degeneracy of the genetic code, also code for the given amino acid sequence.

The term "nucleic acid molecule" also includes analogues of DNA and RNA, such as those containing modified backbones.

In another aspect of the invention, the antigens of the present invention may be prepared by any other method such as SPS (solution phase synthesis) or SPPS (solid phase peptide synthesis). Several ligation methods can be also used for the synthesis of the antigens of the present invention such as chemical ligation, native chemical ligation (NCL), expressed protein ligation (EPL), and Staudinger ligation. Additionally, the O-acyl isopeptide, also known as 'click' or 'switch' peptide, method is useful for the chemical assembly of highly aggregation-prone polypeptides. Othermolecular or genetic engineering techniques, could be used.

Another aspect of the invention is the use of antigens of the invention described above as medicaments, pharmaceutical compositions or the use of the antigens of the present invention in therapy. In particular, one of the aspects of the invention is the use of the antigens of the present invention as active ingredients for the preparation of a medicament or pharmaceutical composition for the prevention and/or treatment of *Toxoplasma gondii* infections.

In the case of gene therapy another object of the invention is the use of the nucleotide sequences coding for the antigens of the present invention as medicaments, in particular for the preparation of medicaments useful for the treatment and prevention of *Toxoplasma gondii* infections.

The pharmaceutical compositions described in the present invention should preferably comprise a therapeutically effective amount of the antigens of the invention or the corresponding nucleotide sequence. Preferably, the antigens of the present invention, and any composition comprising the same, may act or be used as vaccines for the prevention and/or the treatment of *Toxoplasma gondii* infection.

For the therapeutic application, where the preparation of medicaments or vaccines comes within the framework of general knowledge for further reference the reader is again referred to the patent literature cited in the present description and, particularly, to Beghetto et al., The Journal of Infectious Diseases, 2005, 191:637-645.

The term "therapeutically effective amount" as used herein refers to an amount of a therapeutic agent needed to treat, ameliorate, or prevent a targeted disease or condition, or to exhibit a detectable therapeutic or preventative effect. For any compound, the therapeutically effective dose can be estimated initially either in cell culture assays, for example, of neoplastic cells, or in animal models, usually mice, rabbits, dogs, or pigs. The animal model may also be used to determine the appropriate concentration range and route of administration. Such information can then be used to determine useful doses and routes for administration in humans.

The precise effective amount for a human subject will depend upon the severity of the disease state, general health of the subject, age, weight, and gender of the subject, diet, time and frequency of administration, drug combination (s), reaction sensitivities, and tolerance/response to therapy. This amount can be determined by routine experimentation and is within the judgement of the clinician.

A pharmaceutical composition may also contain a pharmaceutically acceptable carrier, for administration of a therapeutic agent. Such carriers include antibodies and other polypeptides, genes and other therapeutic agents such as liposomes, provided that the carrier does not itself induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

Pharmaceutically acceptable salts can be used therein, for example, mineral acid salts such as hydrochlorides, hydrobromides, phosphates, sulphates, and the like; and the salts of organic acids such as acetates, propionates, malonates, benzoates, and the like. A thorough discussion of pharmaceutically acceptable carriers is available in Remington's Pharmaceutical Sciences (Mack Pub. Co., N. J.1991).

Pharmaceutically acceptable carriers in therapeutic compositions may additionally contain liquids such as water, saline, glycerol and ethanol. Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The pharmaceutical compositions utilised in this invention may be administered by any number of routes including, but not limited to, oral, intravenous, intramuscular, intra- arterial, intramedullary, intrathecal, intraventricular, transdermal or transcutaneous applications (for example, see WO98/20734), subcutaneous, intraperitoneal, intranasal, enteral, topical, sublingual, intravaginal or rectal means. Gene guns or hyposprays may also be used to administer the pharmaceutical compositions of the invention. Typically, the therapeutic compositions may be prepared as injectables, either as liquid solutions or suspensions; solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion.

Dosage treatment may be a single dose schedule or a multiple dose schedule.

The method of treating a mammal suffering from *Toxoplasma-gondii* infection, preferably form acute *Toxoplasma-gondii* infection, comprising administering a therapeutically effective amount of the antigens of the present invention, by using for example a vaccine composition as described above, represents one of the aspects of the present invention.

A further aspect of the present invention refers to the *in vitro* use of the antigens of the present invention for detecting anti-*T*. *gondii* IgMs, in particular, for detecting anti-*T*. *gondii* IgMs in a test sample, preferably a biological sample such as whole blood or part of whole blood, e.g., plasma or serum sample. A preferred embodiment refers to the *in vitro* use of the antigens of the present invention as described above for the diagnosis of acute toxoplasmosis in a subject in need thereof, preferably in a human subject.

A still further object of the present invention refers to a kit suitable for detecting anti-*T*. *gondii* IgMs in a test sample, preferably a biological sample such as whole blood or part of whole blood, e.g., plasma or serum sample comprising at least one antigen according to the present invention. Such kits are useful for the diagnosis of an acute *Toxoplasma gondii* infection.

The antigens of the present invention may be employed in virtually any assay format that employs the antigen to detect IgM antibodies from *Toxoplasma gondii* infection. A common feature of all of these assays is that the antigen is contacted with the body component suspected of containing IgM antibodies under conditions that permit the antigen to bind to any such IgM antibody present in the component. Such conditions, in a non limiting manner, can typically be physiologic temperature, pH and ionic strength using-an excess of antigen. The incubation of the antigen with the specimen is followed by detection of immune complexes comprised of the antigen.

Design of the immunoassays is subject to a great deal of variation, and many formats are known in the art. Protocols may, for example, use solid supports, or immunoprecipitation. Most assays involve the use of labelled antibody or polypeptide; the labels may be, for example, enzymatic, fluorescent, chemiluminescent, radioactive, or dye molecules. Assays which amplify the signals from the immune complex are also known; examples of which are assays which utilize biotin and avidin, and enzyme labelled and mediated immunoassays, such as ELISA assays.

The immunoassay may be, without limitation, in a heterogenous or in a homogeneous format, and of a standard or competitive type. In a heterogeneous format, the polypeptide, in particular the antigen of the present invention, is typically bound to a solid matrix or support to facilitate separation of the sample from the polypeptide after incubation.

Examples of solid supports that can be used are nitrocellulose (e.g., in membrane or microtiter well form), polyvinyl chloride (e.g., in sheets or microtiter wells), nanoparticles and microparticles of different compositions (metal selected from iron, gold, platinum, silver, titanium, zinc, cerium, iron, copper, thallium, and combinations thereof; organic, metalorganic, polymeric, quantum dots, or carbon structures), polymers selected from latex, polystyrene, alginic acid, gelatin, polylactic acid, chitosan, polylactide-co-glycolide, and polycaprolactone (e.g., in beads or microtiter plates, polyvinylidine fluoride (known as Immulon^{™}), diazotized paper, nylon membranes, activated beads, and Protein A beads. For example, Dynatech lmmulon^{™}1 or lmmulon^{™}2 microtiter plates or 0.25 inch polysterene beads (Precision Plastic Ball) can be used in the heterogeneous format. The solid support containing the antigenic polypeptides is typically washed after separating it from the test sample, and prior to detection of bound antibodies. Both standard and competitive formats are known in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of antibodies forming the antibody-antigen complex is directly monitored. This may be accomplished by determining whether labelled anti-xenogenic (e.g., anti-human) antibodies which recognize an epitope on *anti-Toxoplasma gondii* antibodies will bind due to complex formation. In a competitive format, the amount of antibodies in the sample is deduced by monitoring the competitive effect on the binding of a known amount of labelled antibody (or other competing ligand) in the complex.

Complexes formed comprising *anti-Toxoplasma gondii* antibody (or, in the case of competitive assays, the amount of competing antibody) are detected by any of a number of known techniques, depending on the format. For example, unllabeled antibodies in the complex may be detected using a conjugate of antixenogeneic Ig complexed with a label, (e.g., an enzyme label).

In an immunoprecipitation or agglutination assay format the reaction between the chimeric antigens and the antibody forms a network that precipitates from the solution or suspension and forms a visible layer or film of precipitate. If no *anti-Toxoplasma gondii* antibody is present in the test specimen, no visible precipitate is formed. Example of an agglutination immunoassay is a turbidimetric assay using a suspension of polystyrene particles of uniform size coated with the antigens of the present invention. When a sample containing IgMs against *Toxoplasma gondii* is mixed with the reagent, the coated latex particles agglutinate. The degree of the agglutination is directly proportional of the concentration of the IgMs in the sample and is determined by measuring the decrease of transmitted light caused by the aggregates (turbidimetric immunoassay). Another example of an immunoassay is a two-step chemiluminescence assay consisting of magnetic particles coated with the antigens of the present invention. After incubation with a sample, magnetic separation, and a wash step, beads are incubated with an isoluminol-labeled anti-human IgM antibody. After a final wash, reagents that trigger the luminescence reaction are added, and the emitted light is measured as relative light units (RLUs). The RLUs are directly proportional to the Toxo anti-IgM concentration in the sample.

The antigens of the present invention will typically be packaged in the form of a kit for use in these immunoassays. The kit will normally contain in separate containers the combination of antigens (either already bound to a solid matrix or separate with reagents for binding them to the matrix), control antibody formulations (positive and/or negative), labelled antibody when the assay format requires same and signal generating reagents (e.g., enzyme substrate) if the label does not generate a signal directly. Instructions for carrying out the assay usually will be included in the kit.

The invention will now be illustrated in greater detail by means of examples and figures.

### Brief description of the figures

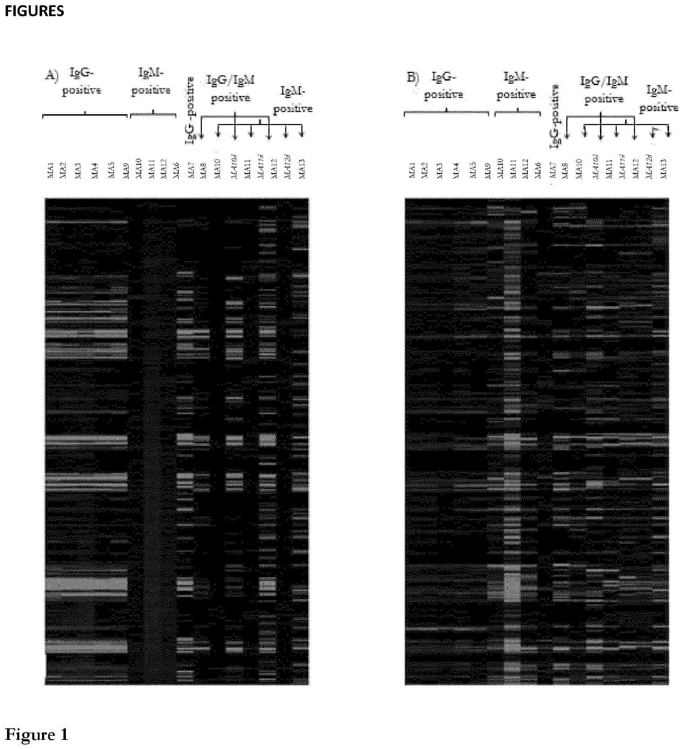

**Figure. 1****.** Raw heatmaps of *T. gondii* peptide microarray. The heatmaps are represented on a grey color code: grey for strong and black for weak or no antibody responses. (A) IgG response heatmap using goat antihuman IgG (Fc) fluorescent secondary antibody and (B) IgM response heatmap using goat anti-human IgM (µ chain) fluorescent secondary antibody. (PEPperPRINT GmbH source).

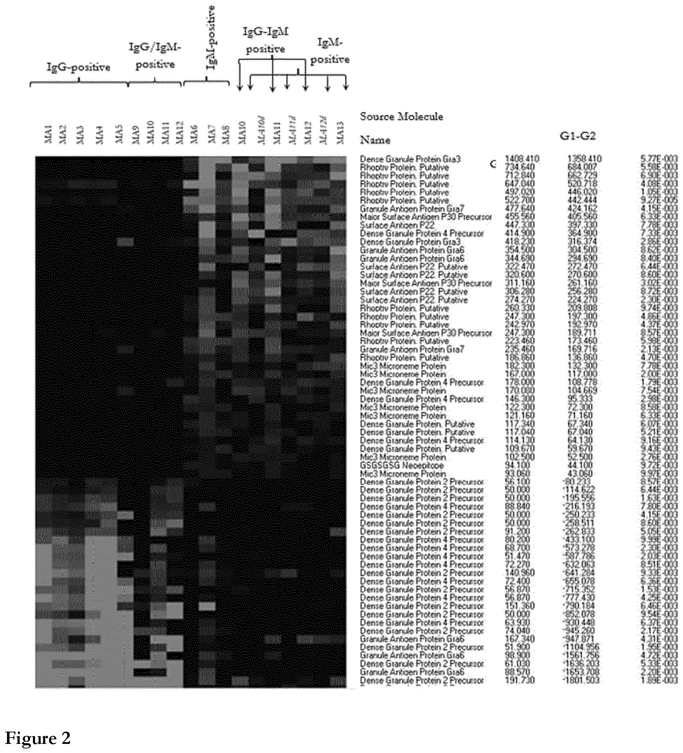

**Figure 2****:** *T*. *gondii* peptide microarray heatmap showing unpaired two-class t-test of differential IgM response. Source molecule name: protein to which each peptide belongs (sequence not shown). G1- Mean: mean value of all IgM-positive samples. G1-G2 difference: the result of the two-class t-test. Peptides are sorted by decreasing G1-G2 differences.

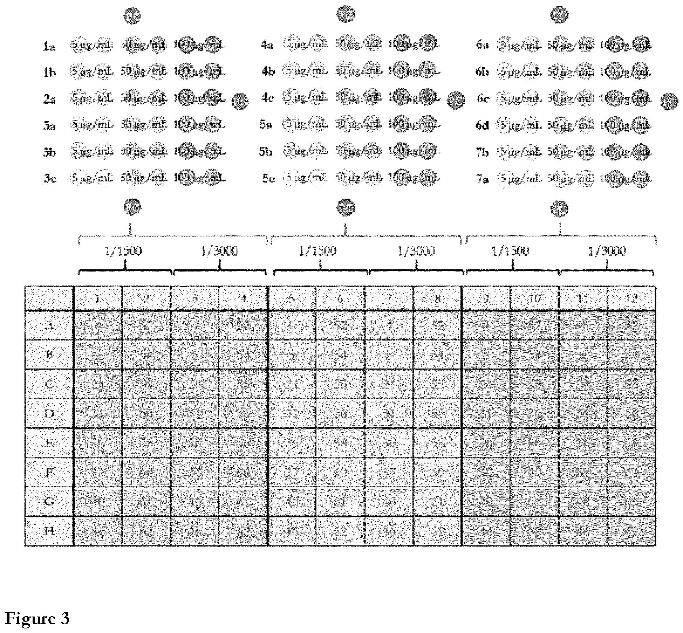

**Figure 3****:** Schematic view of the multiplex ELISA. The upper panel represents the spotting pattern within each section of the 96-well ELISA plate (1 to 3 upper row of the ELISA plate) represented in the lower panel. The first four columns of the ELISA plate were printed with peptides 1a to 3c, Columns 4 to 7 were printed with peptides 4a to 5c, and columns 8 to 12 were printed with peptides 6a to 7b. The eight positive and negative samples were evaluated following the pattern shown in the picture. PC=positive control.

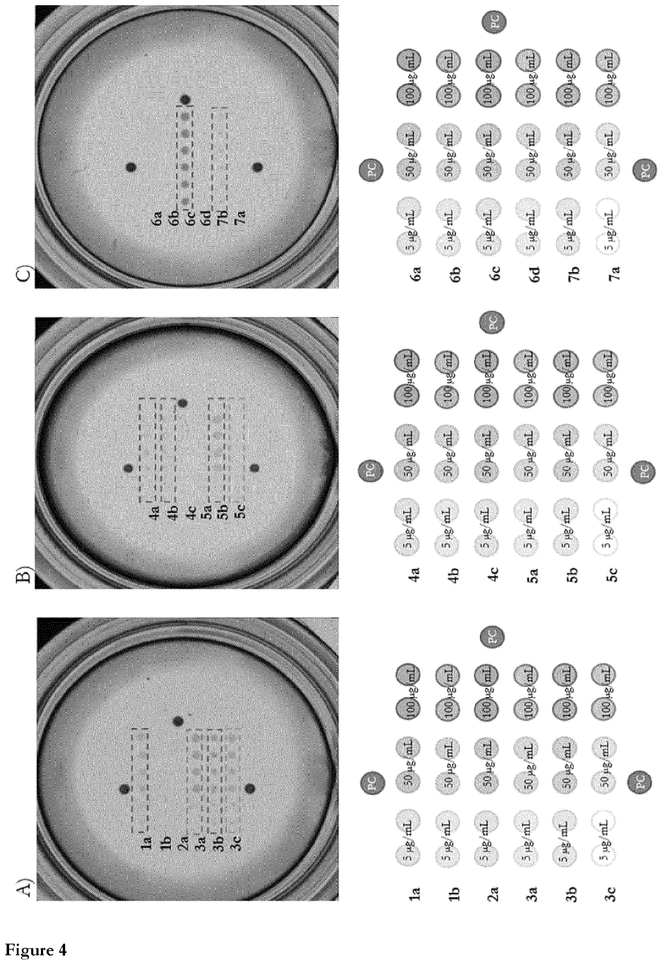

**Figure 4****:** Scanned images for sample 4 with the 18 peptides. Panel a) corresponds to scanned images of peptides 1a to 3c. Panel b) corresponds to scanned images of peptides 4a to 5c. Panel c) corresponds to scanned images of peptides 6a to 7a. A schematic view of the spotting pattern of each well is showed in the right side of each panel. The reactivity of peptides which showed S/CO ≥ 2.2 is highlighted with a hyphen-line figure. (PC: positive control).

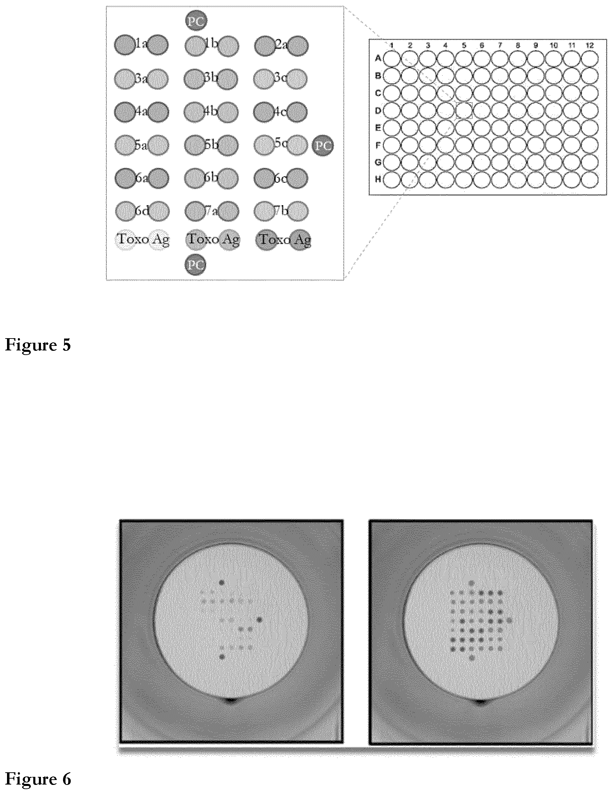

**Figure 5****:** Schematic view of the multiplex ELISA of phase II. Each well of two 96-well plates was printed with the 7x6 spotting matrix represented in the figure. (PC: positive control, *Toxoplasma* Ag: native antigen of *T. gondii*)*.*
**Figure 6****:** Original image for positive sample 4, before and after processing. Left image before analysis. Right image after software processing.

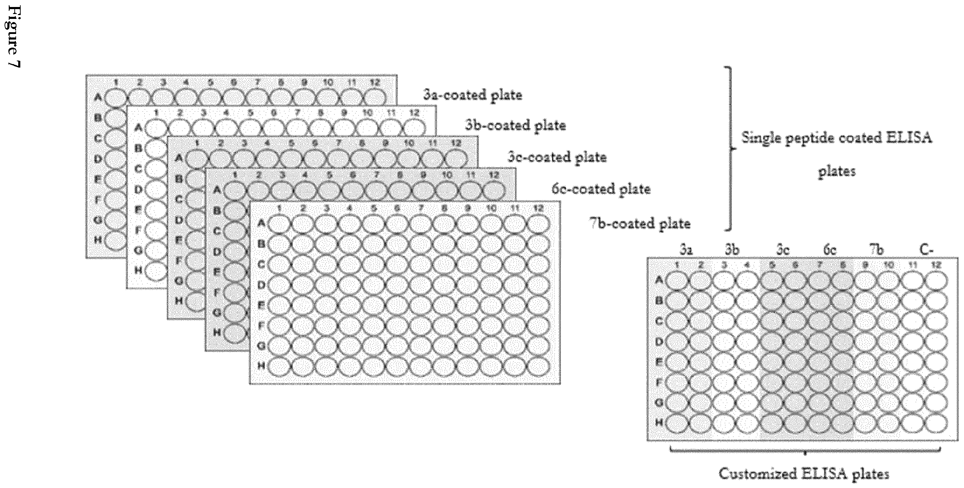

**Figure 7****:** Schematic view of ELISA plates. ELISA plates coated with a single peptide were initially produced in single strip-detachable 96-well plates. Ready-to-use, customized ELISA plates were easily obtained using strips from the original plates on demand. C- strips refers to control strips, for which we followed the same coating procedure but without peptide.

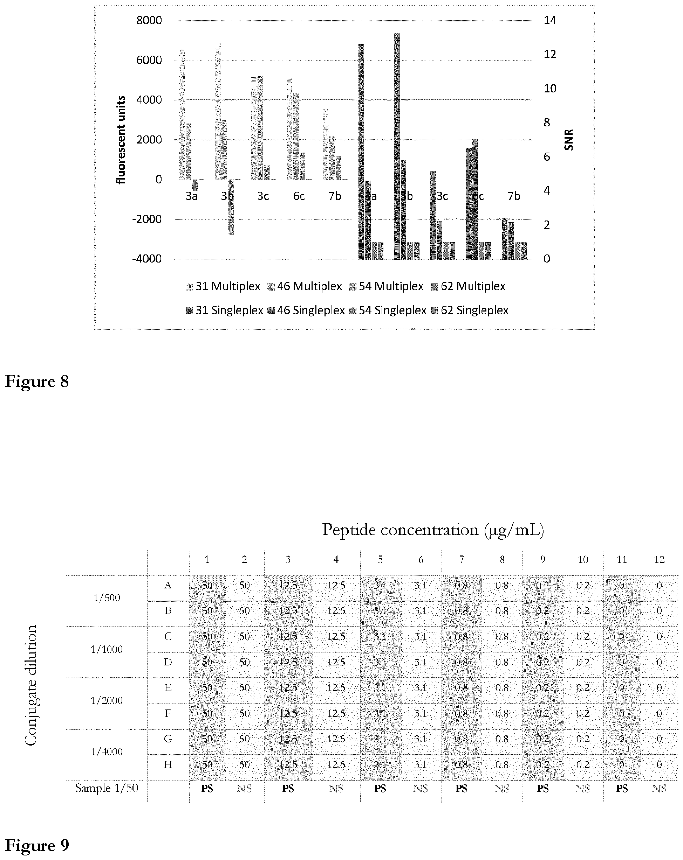

**Figure 8****:** Correlation between multiplex and singleplex ELISA. Positive samples (31 and 46) and negative samples (54 and 62) are represented for each technique with their corresponding units (fluorescent units and signal-to-noise ratio (SNR) respectively. Al peptides were tested at 100µg/mL.
**Figure 9****:** Example of a checkerboard template. Every two columns the peptide concentration was reduced 1:4. The two last columns are used as a negative control. Every two rows the conjugate was diluted 1:2. One column of each condition is used to test a positive sample (PS) and the other to test a negative sample (NS). The sample dilution is fixed at 1/50 with sample diluent.

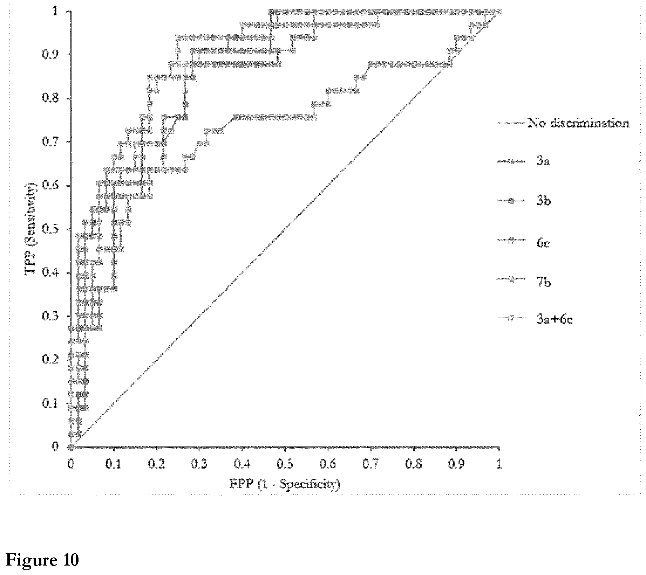

**Figure 10****:** Receiver operating characteristic curve of peptides 3a, 3b, 6c 7b and 3a+6c. The diagnostic performance of each peptide is represented by a different grey intensity line. The solid grey line indicates the non-discrimination area of the ROC curve. The conditional sample panel, minus sample 49 was used to generate the curve (n=93).
**Figure 11****:** Schematic representation of original and truncated version of peptide 6c. 6c peptide consisted of the tandem repetition of three blocks of eight amino acids plus two amino acids in the N-terminus, and one amino acid in the C-terminus (27aa). Truncated 6c peptide consisted of the same block tandem repetition minus two amino acids in the N-terminus (25 aa). Each different amino acid within the block is named with a different letter. O, O' and O" represent the single amino acid that changes within the block.

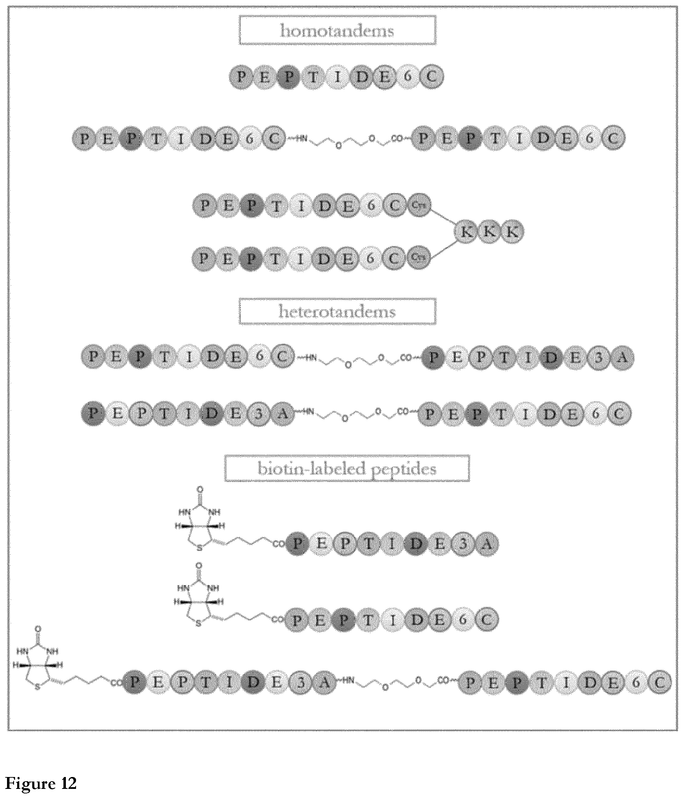

**Figure 12****:** Schematic representation of the chimeric and biotin-labelled peptides. 6c homotandems consisted in tandem repetition of the 6c peptide in three different formats. 3a and 6c heterotandems consisted of tandem repetition of 3a and 6c sequences using O2Oc as a spacer, in different orientations. 3a and 6c truncated peptide were biotin-labelled.

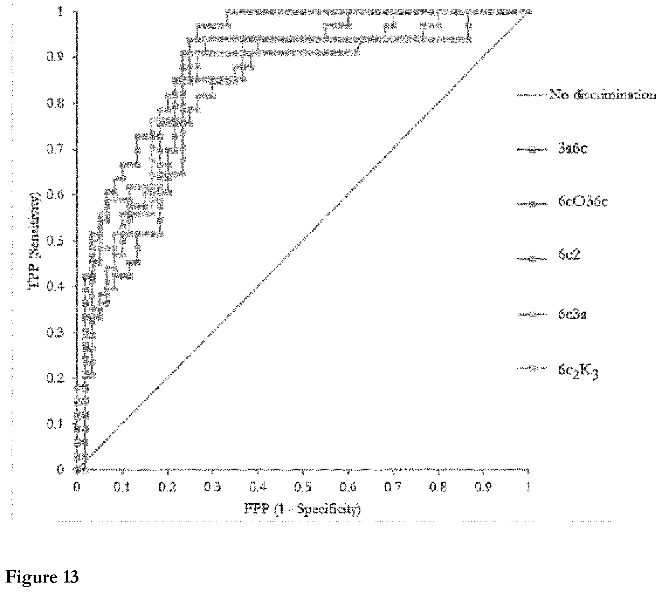

**Figure 13****:** Receiver operating characteristic curve of chimeric peptides. The diagnostic performance of each peptide is represented by a different grey intensity line. The solid grey line indicates the non-discrimination area. The conditional sample panel, minus samples 49 and 31 (n=92) were used to generate the ROC curve for peptides 6c2, 6cO36c and 3a6c. For peptides (6c2)K3 and 6c3a, the conditional sample panel, minus sample 31, (n=93) was used.

### Examples

### Example 1. Synthetic peptides as antigens: from T. gondii peptide microarray to functional individual peptides

As already mentioned in the background of the present invention, discovering new epitopes that could help in the clinical management of toxoplasmosis is one of the foci of this invention. Identifying which proteins of the *T. gondii* parasite could be used as new potential biomarkers for the diagnosis of the acute phase of the disease is our starting point, thus a first objective of the present invention is to study the main antigenic proteins of *T. gondii.* Given the drawbacks of *T. gondii* lysate antigens and recombinant product-based assays, we opted for a peptide-based approach aimed at identifying epitope sequences with potential application in an immunoassay for anti-*T*. *gondii* IgM detection. To this end, we first used an epitope mapping technology involving a peptide microarray developed by PEPperPRINT GmbH (Heidelberg, Germany) to find new antigens specific for IgM and with no or little reactivity to other Ig types (i.e. IgG).

In a subsequent step, we used solid phase peptide synthesis (SPPS) to produce scaled-up, high-quality synthetic versions of relevant sequences identified in the PEPperPRINT scan as free (i.e., not microarray-bound) soluble peptides to be incorporated into diagnostic kits. Finally, we evaluated the antigenic capacity of the peptides using an ELISA. Initially, we developed a multiplex ELISA to evaluate the feasibility of synthetic peptides for detecting anti-*T*. *gondii* IgM. Once we identified the best peptides, we used a singleplex ELISA to validate the results obtained by the multiplex ELISA. Finally, the singleplex ELISA was optimized to improve the performance of the assay to detect anti-*T*. *gondii* IgM-positive samples.

### 1.1. T. gondii peptide microarray

To identify new *T. gondii* epitopes that could be incorporated as diagnostic tools in an immunoassay for detecting anti-*T*. *gondii* IgM, we initiated a collaboration with PEPperPRINT GmbH to design a peptide microarray that displayed all peptides covering different antigenic proteins from *T. gondii.* A complete list of the nine proteins selected, identified by means of UniProt (https://www.unipro*T*.org/) and the Immune Epitope Database (IEDB) (https://www.iedb.org/), is shown in Table 1.

### 1.2. Microarray sample panel characterization

For a preliminary assessment of the selected proteins in the epitope mapping microarray experiment, a panel of 13 human serum samples (henceforth microarray serum panel) obtained from Cerba Laboratory and Biokit biobank was fully characterized for *T*. *gondii* IgM and IgG immunoreactivity. Different immunoglobulins, in particular IgG and IgM, exhibit distinct profiles and kinetics during toxoplasmosis, so it was essential to ascertain which antibody types were present in the microarray serum panel. Due to the high prevalence of the disease, *T*. *gondii* IgG-positive samples lacking IgM are the more frequent type observed in clinical routine. In contrast, *T*. *gondii* IgM-positive samples devoid of IgG, characteristic of initial phases of the disease, are difficult to obtain. This is mainly because early diagnosis of toxoplasmosis is unusual, since immunocompetent patients remain asymptomatic in most cases, or the clinical picture is complex and can be confused with a variety of other diseases. Therefore, the most usual scenarios are that either both anti-*T*. *gondii* IgG and IgM are normally found together, or IgG alone is detected.

For our microarray evaluation it was essential that samples containing only IgM were adequately represented. To this end, three samples (MA10, MA11 and MA12) were IgG-depleted to remove anti-*T*. *gondii* IgG; thus, depleted and non-depleted samples were included for the microarray evaluation.

Characterization of the microarray sample panel was done by means of the commercial BIO-FLASH^{®} Toxo IgG and the BIO-FLASH^{®} Toxo IgM immunoassays. Results are shown in Table 3.

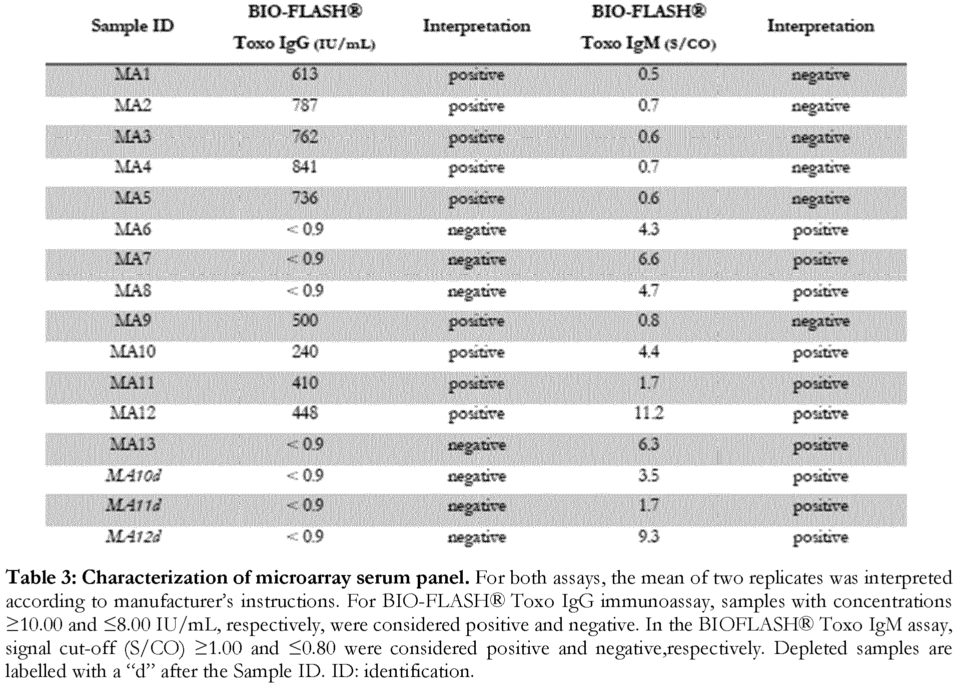

Analysis of the microarray serum panel revealed that six samples were positive according to the BIOFLASH^{®} Toxo IgG immunoassay and negative according to the BIO-FLASH^{®} Toxo IgM (MA1 to MA5 and MA9), four samples were positive for IgM and negative for IgG (MA6 to MA8 and MA13), and three samples were IgG-and IgM-positive (MA10 to MA12). Depleted samples (MA10d to MA12d) showed 99% IgG titer reduction relative to the non-depleted samples. Therefore, they were classified as IgG-negative. The depletion process caused a 17-20% reduction in IgM reactivity compared to non-depleted ones.

### 1.3. T. gondii peptide microarray evaluation.

The sequences of the nine selected *T. gondii* proteins were evaluated by PEPperPRINT GmbH, in a peptide microarray vs. the microarray serum panel. Goat anti-human IgG (Fc) fluorescent conjugated DyLight680 and goat anti-human IgM (µ chain) fluorescent conjugated DyLight800 were used as secondary antibodies to differentiate either type of antibody response in each group of samples. IgG and IgM heatmaps of the unprocessed fluorescent images are presented Figure 1.

The IgG response heatmap shows that IgG-positive samples (MA1 to MA5) had a similar fluorescent signal profile and intensity. For their part, IgG-negative samples (MA6 to MA8) exhibited no unspecific binding to any epitopes evaluated, altogether highlighting a clear difference between both groups of samples. As expected, depleted samples (MA10d, to MA12d) showed no response for IgG compared with non-depleted ones (MA10, to MA12), which were positive for some epitopes. Sample MA13, despite being initially classified as IgM-positive, showed no differences between both IgG and IgM peptide microarrays and was thus considered an outlier, not included in the statistical analysis (Figure 1A).

The IgM response heatmap revealed a more complex profile than that of the IgG. IgM-positive samples, regardless if IgG-positive or negative (MA6 to MA8, MA10 to MA12 and MA10d to MA12d), demonstrated a distinctly stronger response than IgM-negative ones (MA1 to MA5 and MA9). Sample MA7 had the highest reactivity within the IgM-positive group, consistent with the results from the previous BIO-FLASH^{®} Toxo IgM (6.6 S/CO). In contrast, samples MA12 and MA12d, with high IgM titers in the BIO-FLASH^{®} Toxo IgM characterization (11.2 and 9.3 S/CO respectively), showed weaker reactivity (Figure 1B).

For data analysis, the microarray serum panel was classified into four different groups as follows: Group 1 (G1): IgM-positive (MA6, MA7, MA8, MA10, MA11, MA11d, MA12, MA12d and MA13); G2: IgG-positive (MA1, MA2, MA3, MA4, MA5, MA9, MA10, MA11 and MA12); G3: IgG-negative (MA6, MA7, MA8, MA10d, MA11d and MA12d) and G4: IgM-negative (MA1, MA2, MA3, MA4 and MA5).

The results obtained were statistically analysed using the Statistical Utility for Microarray and Omics data (SUMO) software (Heidelberg, Germany), based on the unprocessed averaged spot intensities without data normalization. As a noise filter, a minimum spot intensity of 50 fluorescence units was defined, (i.e., all interactions below the threshold were set to 50 and had no influence on the statistical analysis). Unpaired two-class t-test were performed to identify: (i) common IgG responses (IgGpositive against IgG-negative samples), (ii) common IgM responses (IgM-positive against IgMnegative samples) (data not shown), and (iii) differential IgM (henceforth G1-G2) (IgM-positive against IgG-positive samples).

To elucidate which epitopes were specific for IgM, differential IgM responses were evaluated. Averaged spot intensities of IgM-positive sera (MA6, MA7, MA8, MA10, MA11, MA11d, MA12, MA12d and MA13) vs. IgG-positive sera (MA1, MA2, MA3, MA4, MA5, MA9, MA10, MA11 and MA12) were distributed in a response matrix that included 82 peptides with a p-value of p < 1.00E-02. From 82 peptides, up to 39 were statistically significant and thus upregulated in the IgM-positive vs. IgG-positive samples. Based on the information in the differential IgM response heatmap, top peptide hits exhibited G1-G2 differences of >400 units, which were predominantly assigned to dense granule protein GRA3, rhoptry protein, granule antigen protein GRA7 and major surface antigen P30 (Figure 2).

A detailed view of the two-class t-test analysis performed on the 21 statistically significant peptides previously seen in Figure 2, which exhibited the highest G1/G2 ratios, is shown in Table 4. The top upregulated peptides (peptides I to VIII) showed G1-G2 differences of >400 units and almost all showed G1/G2 ratios of >5, which indicated that they were 5-fold more reactive for the IgM positive samples (G1) in comparison with the IgG-positive samples (G2). The same peptides presented low reactivity for IgG-positive samples, G2 between 50 and 126, which highlighted their specificity for IgM-positive samples.

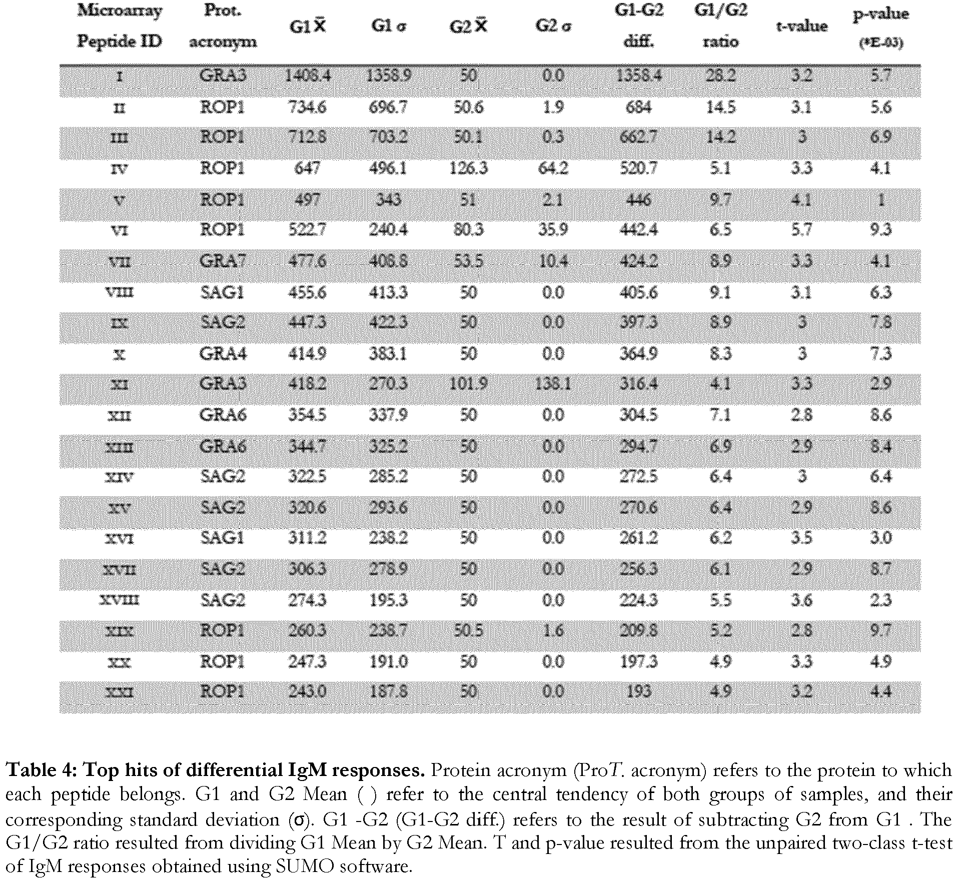

### 1.4. From microarray to synthetic peptides

The drawbacks of TLA or recombinant antigens contrast with the proven advantages of synthetic constructs, such as minimizing non-specific interactions, higher reproducibility and the possibility of adapting their chemical structure to enhance their antigenic properties. Therefore, we chose an approach based on synthetic peptides to reproduce the sequences identified in the above screening.

Based on the results of the microarray analysis, we identified the sequences that had the potential to perform best. We based our selection on the following: i) IgM specificity based on G1-G2 differences and G1/G2 ratio for differential IgM responses, ii) homology to *T. gondii* B cell epitopes found in IEDB, and iii) peptide length. Thus, we designed the peptides to include the areas within the studied proteins that were most specific for IgM but limiting peptide length to around 50-60 residues, which is the approximate threshold after which the quality of synthetically produced peptides could be compromised. The 18 selected peptides, were synthesized in C-terminal carboxamide form using Fmoc solid phase synthesis, purified to >95 % homogeneity using HPLC (high performance liquid chromatography) and satisfactorily characterized using MS (mass spectrometry) (see Table 2).

### 1.5. Evaluation of synthetic peptides via ELISA

Once the new synthetic peptides were produced, we wanted to evaluate their functionality using both multiplex and singleplex indirect ELISA. To evaluate the selected peptides, a panel of 110 human sera (hereafter screening panel) was acquired from several suppliers. AbBaltis, ABO Pharma, and Biomex as external vendors provided *T. gondii* IgM-positive samples (n=46). Two IgM-positive samples from the microarray panel were included to verify the results obtained by the peptide microarray (MA10, and MA11). Sixty samples that came from Banc de Sang i de Teixits de Catalunya were acquired in two different batches as *T. gondii* IgM negative.

To preliminary study cross-reactivity, Biokit biobank provided two supplementary *T. gondii* negative samples that were positive for cytomegalovirus (CMV), as this virus was described by other commercial assays as a possible cross-reactant. The composition of the sample panel before the internal characterization can be seen in Table 5.

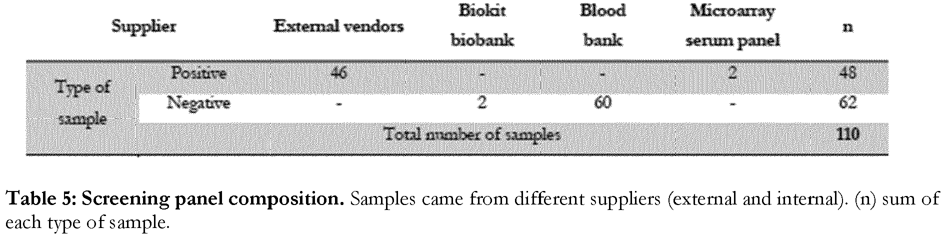

Samples provided by external vendors were previously characterized for *T. gondii* IgM using different commercial assays (data not shown). The two negative samples provided by Biokit biobank were classified as negative according to previous serologic information (data not shown). Samples acquired from the blood bank were not previously tested for toxoplasmosis infection. The three IgM-positive samples belonging to the microarray panel were tested within this invention. To validate the results provided by the suppliers and the agreement between IgM anti-*T*. *gondii* detection methods, the screening panel was fully characterized for the presence of IgM by means of four commercial assays that were selected considering i) assay performance (high sensitivity and specificity), ii) system requirements (i.e., methodology or specific instrumental requirements) and iii) the capacity to obtain reagents and technical support. BIO-FLASH^{®} Toxo IgM immunoassay (Biokit), bioelisa TOXO IgM (Biokit) and PLATELIATM Toxo IgM (BIO-RAD) were selected. Additionally, VIDAS^{®} TOXO Competition (BioMérieux) for the detection of total anti-*T*. *gondii* Ig was included as a preliminary screening method (Table 6).

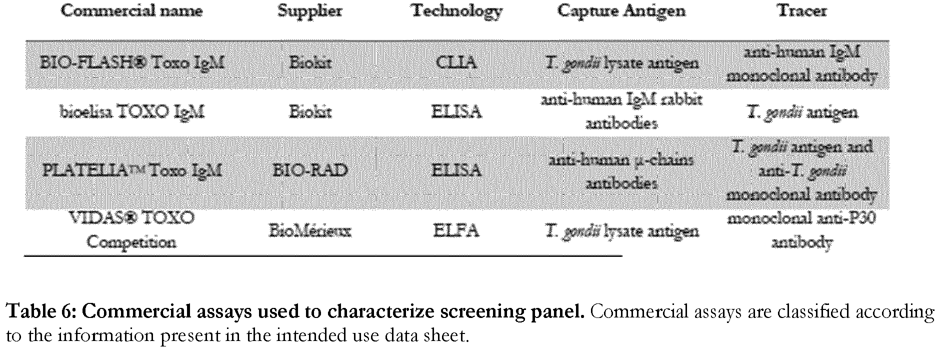

To characterize the screening panel, we designed a strategy to prioritize analysing positive samples from external vendors (n=46) using the four assays (BIO-FLASH^{®} Toxo IgM, bioelisa TOXO IgM, PLATELIATM Toxo IgM and VIDAS^{®} TOXO Competition). Finally, from the two microarray serum panel samples, due to the available volume of each sample, we tested MA10 and MA11 samples with only two commercial assays. The 60 samples provided by the blood bank, which we expected to be mostly negative, were tested using one or two assays considering both the sample volume and the assays available in-house when the samples were received. When a result was positive, we expanded the screening using a second or third assay. The first batch consisted of 20 samples that were tested using two assays (blood bank batch I). From those 20 samples, one gave a positive result; thus, it was evaluated by a third assay, which gave a negative result. In the second batch 40 samples were received and tested using two assays (blood bank batch II). From those, one gave a positive result; hence it was tested using a third assay, which gave a negative result. Table 7 details the results of the screening panel characterization.

The results we obtained with the 46 samples purchased as IgM anti-*T*. *gondii* positive varied among the four commercial assays used to characterize the samples, ranging from 45 to 30 positives. VIDAS^{®} TOXO Competition was the assay that classified the greatest number of samples as positive (n=45), whereas BIO-FLASH^{®} Toxo classified the lowest number of samples as positive (n=30). Regarding negative samples, the same estimations could not be performed, because not all samples were evaluated by the same assays.

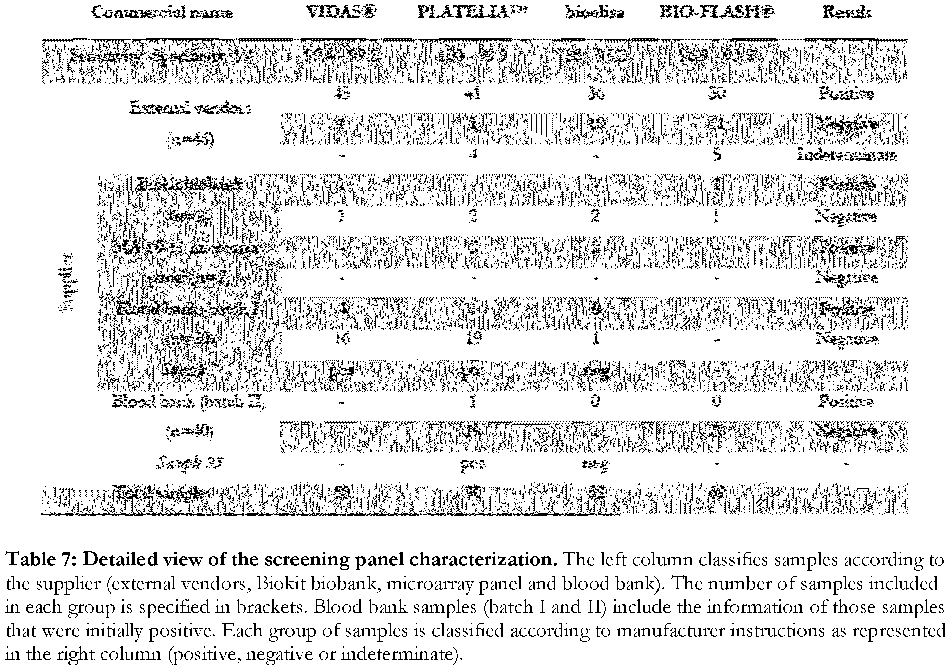

These results show that the four commercial assays did not agree on the positivity of the samples. Since discerning positive from negative samples was key for the present invention, we classified the panel according to the agreement across the assays used to evaluate each sample. The classification is shown in Table 8.

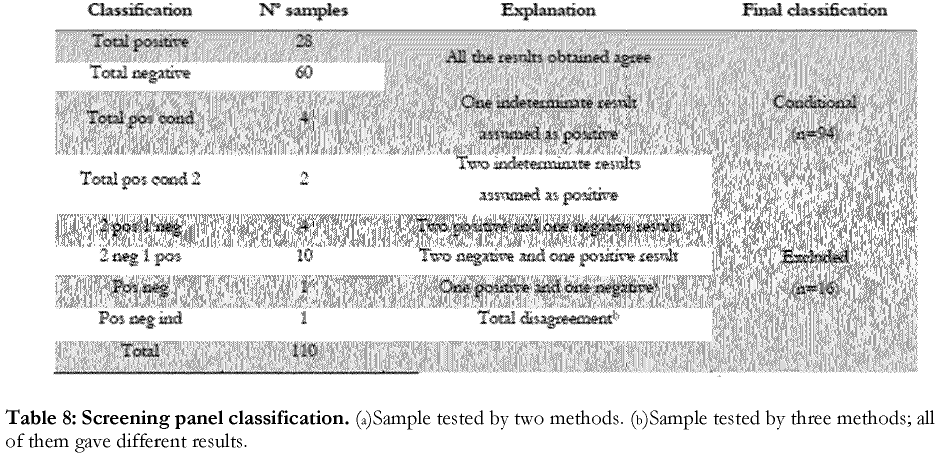

Samples that showed total agreement among all the methods used were classified as total positive (n=28) or total negative (n=60). Samples that were purchased from external vendors as positives, but during our characterization showed one indeterminate result, were classified as total positive conditional (total pos cond) (n=4) or as total positive conditional 2 (total pos cond 2) for samples that presented two indeterminate results (n=2). That group of samples (total positive, total negative and samples with one or two indeterminate results) was named as "conditional" (n=94). The group of samples with final classification as "excluded" (n=16) correspond to samples that showed different degrees of disagreement, either the same number of positive and negative results or even one result of each type (positive, negative and indeterminate). These samples were excluded from the study.

### 1.6. Multiplex ELISA

The microarray study elucidated 18 different peptides that potentially reacted with IgM anti-*T. gondii* positive samples and did not react with anti-*T*. *gondii* IgG-positive samples. The sequences of the selected peptides were synthesized by SPPS. To identify which reacted with the maximum number of IgM-positive samples in a fast and efficient way, and considering that the sample volume was scarce, we designed a multiplex ELISA in collaboration with InfYnity Biomarkers.

Phase I. To elucidate which were the best peptides, we initially needed to design the optimal assay with the appropriate assay conditions. To establish optimal peptide concentration, three peptide dilutions (5, 50 and 100 µg/mL) were spotted in duplicate and organized in a 6x6 array in each well of a 96-well plate (Figure 3). In addition, positive control (PC) was spotted in triplicate to check the reactivity in each well and for array space-orientation. Two different conjugate dilutions were tested (1/1500 and 1/3000). Eight total positive (4, 5, 24, 31, 36, 37, 40 and 46) and eight total negative (52, 54, 55, 56, 58, 60, 61 and 62) samples from the conditional panel were selected and diluted 1/50. The ELISA plate design is shown in Figure 3.

Fluorescent net intensity (mean value of duplicated spots) was established for the 16 samples included in the study. The mean value of negative samples was used to establish individual cut-off for each peptide (Table 9A). Almost all cut-off values were under 600 units. Peptides 6d, 7a and 7b showed higher reactivity (672, 723 and 625, respectively). Two peptides showed cut-off values 2- and 3-fold higher than the others (cut-off of peptide 1a was 1545 and 5b was 3953).

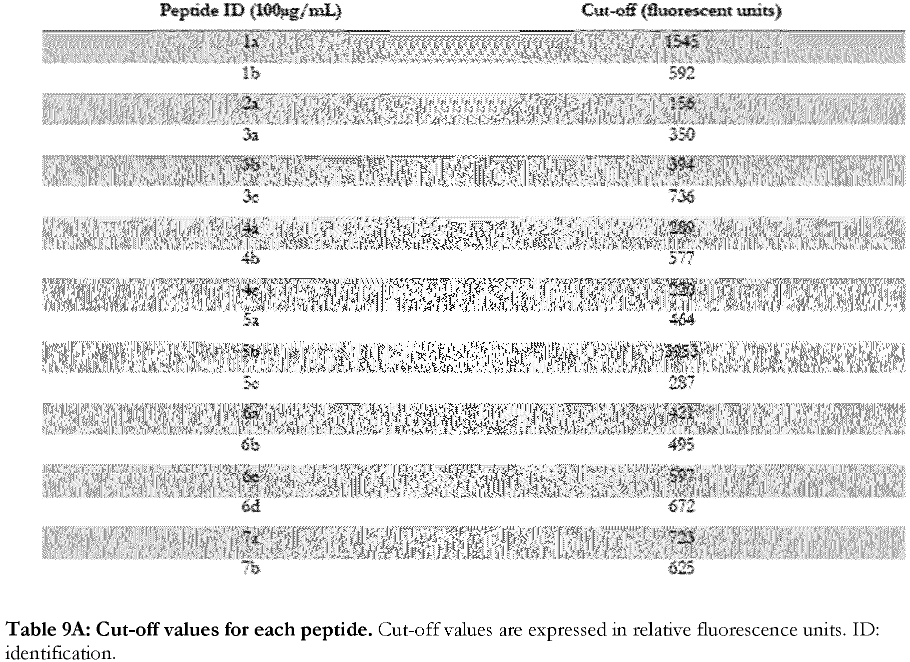

The following table 9B indicates further information in connection to each of the peptides indicated in table 9A above:

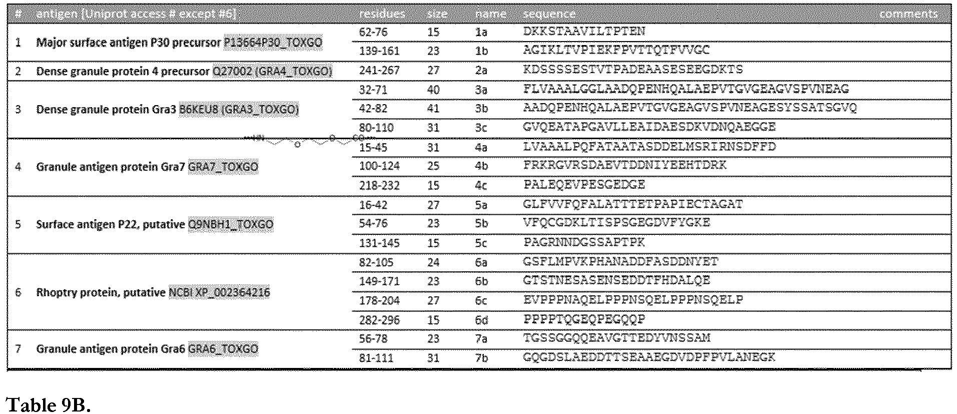

Net intensity of each peptide for positive or negative samples was converted into positive or negative reactivity according to the cut-off value. Signal-to-cut-off value (S/CO) of each sample is presented in Table 10. Also, final S/CO was calculated by subtracting mean negative value from mean positive value. Samples with S/CO values ≥1 were considered positive and samples with S/CO <1 were considered negative. Peptides that showed final S/CO values >1 were considered specific in front anti-*T*. *gondii* IgM-positive samples.

Positive samples were reactive to almost all peptides (1a, 2a, 3a, 3b, 3c, 4a, 4b, 4c, 5a,5b, 5c, 6b, 6c, 6d and 7b). Peptides 3a, 3b, 3c, 6c, and 7b showed higher final S/CO (≥ 3,5). Peptides 5a and 5c showed final S/CO ≥1. The final S/CO for peptide 4a was equal to 1. Peptides 5b, 6a, and 7b reacted more for negative samples compared to positive samples. This outcome suggests that those peptides were non-specific for IgM-positive samples.

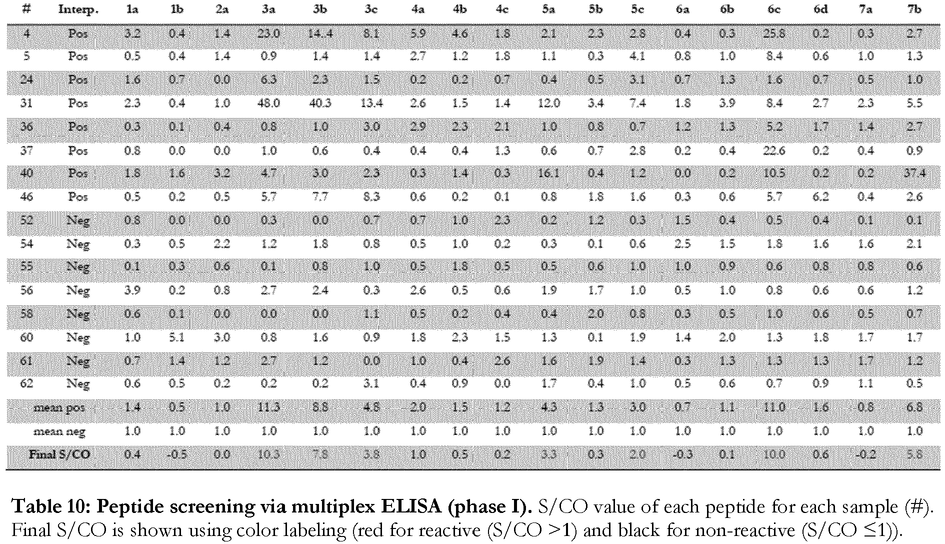

An example is shown below of putting the invention into practice.

Reactivity of 18 peptides for sample 4 can be visually explored in Figure 4 and correlated with numerical cut-off values presented in Table 11. For each peptide, as nonspecific results could be reported when precipitates appeared, all scanned images were visually reviewed and contrasted with their net intensity values. Peptide 6c, one of the best performing (together with 3a), is marked in Figure 4. 6c presented the most intense spots visually, correlating with its S/CO of 25.8, the highest of all peptides (see Table 11). One example of non-specific results is observed when peptides 5b and 5c are compared.

Visually, spot intensities of 5b were higher than 5c. In contrast, cut-off values were higher for peptide 5c (2.8 S/CO vs. 2.3 S/CO, respectively). This example illustrates the importance of comparing the numerical result obtained by the software and prioritizing the visual exploration of the images, to avoid erroneous interpretations of the results. Multiplex ELISA phase I allowed us to establish the optimal assay conditions (peptide concentration at 100 µg/mL, samples at 1/50 and conjugate at 1/1500). The results obtained indicate that peptides 3a and 6c were reactive for IgM anti-*T*. *gondii* for the eight positive samples analysed and nonreactive for the eight negative samples. In contrast, peptides 1a, 5b and 7a presented cross-reactivities with the negative samples which may indicate that they were non-specific for IgM anti-*T*. *gondii.* Even so, the study of the 18 peptides was extended to phase II, with a higher number of samples (the complete sample panel consisting of 110 samples) using the assay conditions established in phase I.

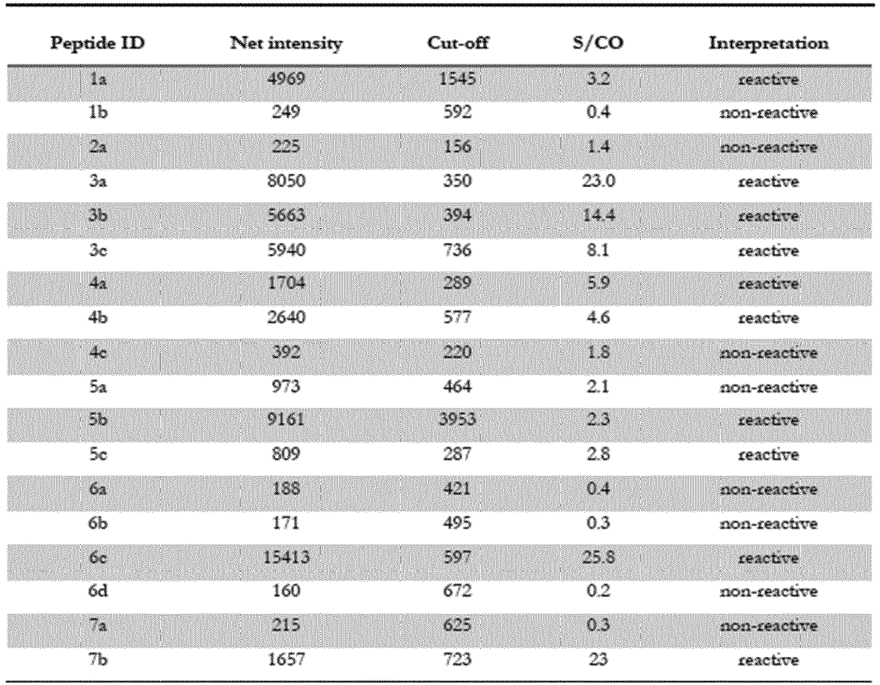

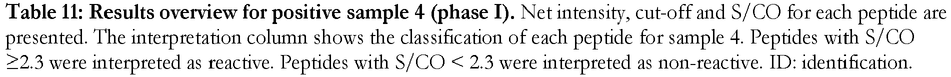

Phase II. As explained before, Phase I allowed us to establish the appropriate assay conditions for the 18 peptides. In phase II, our objective was to identify which peptides had the best antigenic properties. To do so, we analysed the performance of each peptide - the number of samples that were correctly classified as positive or as negative - by using the assay conditions established in phase I with the complete screening panel. Peptides at 100 µg/mL were spotted in duplicate and organized in a 7x6 array in each well of a 96- well plate (Figure 5). Additionally, native antigen of *T. gondii* produced by Biokit was included as an extra control. Biokit Toxo antigen was spotted in duplicate at three different concentrations (5, 50 and 100 µg/mL).

In phase II, InfYnity Biomarkers changed the plate reader and the software used to capture and analyse images from the 96-well plate. The new software establishes the cut-off level according to the data overall. Then, net intensity of each antigen for positive and negative samples was converted into positive or negative reactivity according to the cut-off value established by the software. Although the complete screening panel (n=110) was used, data analysis was performed only on the conditional panel (see Table 8) (samples that were characterized as total positive, total pos cond, total pos cond 2 and total negative) (n=94). Only samples that reported high homogeneity among the assays used to characterize the samples were considered for the analysis, in order to minimize the possibility of including false positives or false negatives, which could have led to data misinterpretation and choice of non-specific peptides. Results are shown in Table 12.

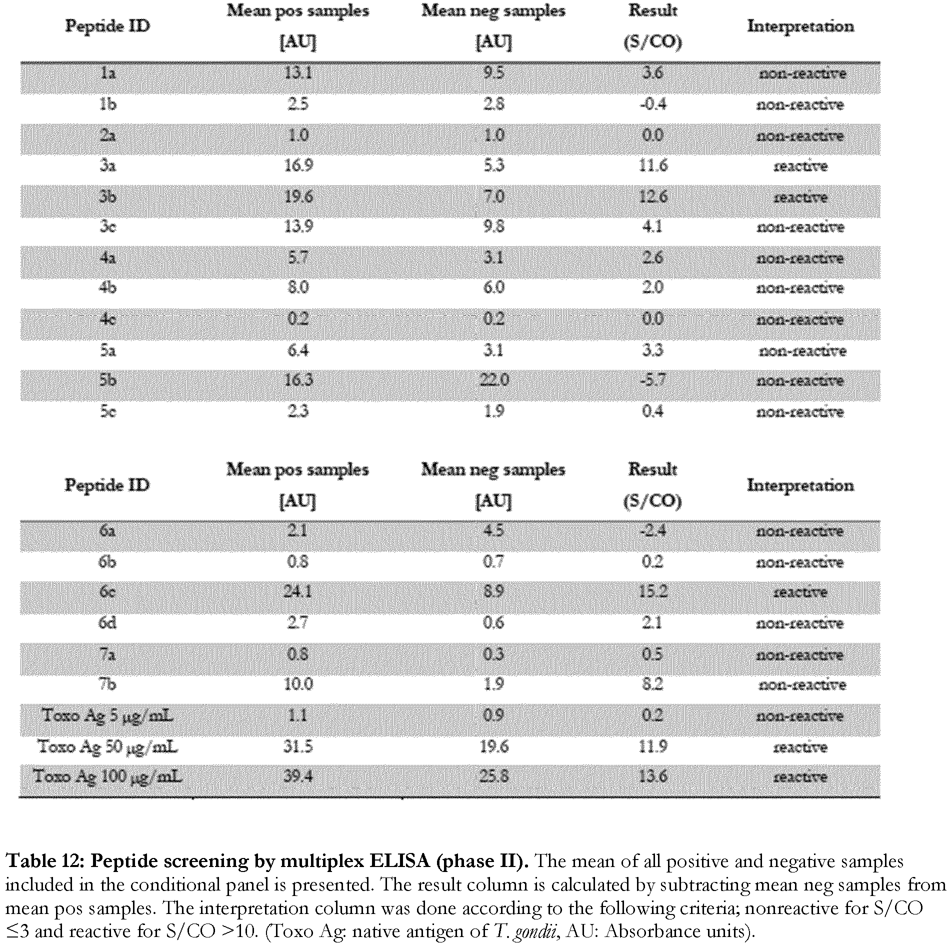

The reactive peptides showing the highest S/CO were 3a, 3b, 6c. Native antigen of *T. gondii* was positive only when tested at 50 and 100 µg/mL. Even so, the mean values of the negative samples were the highest shown in the study, followed by peptide 5b, which may suggest that the use of native antigen of *T. gondii* is not a good approach for detecting IgM anti-*T*. *gondii-*positive samples. A specific example is shown in Figure 6 and Table 13. The original image obtained with sample 4 was processed using the software according to the parameters established. Numerical results were then obtained. Interpretation was provided according to the established cut-off and an indeterminate zone was proposed by the software.

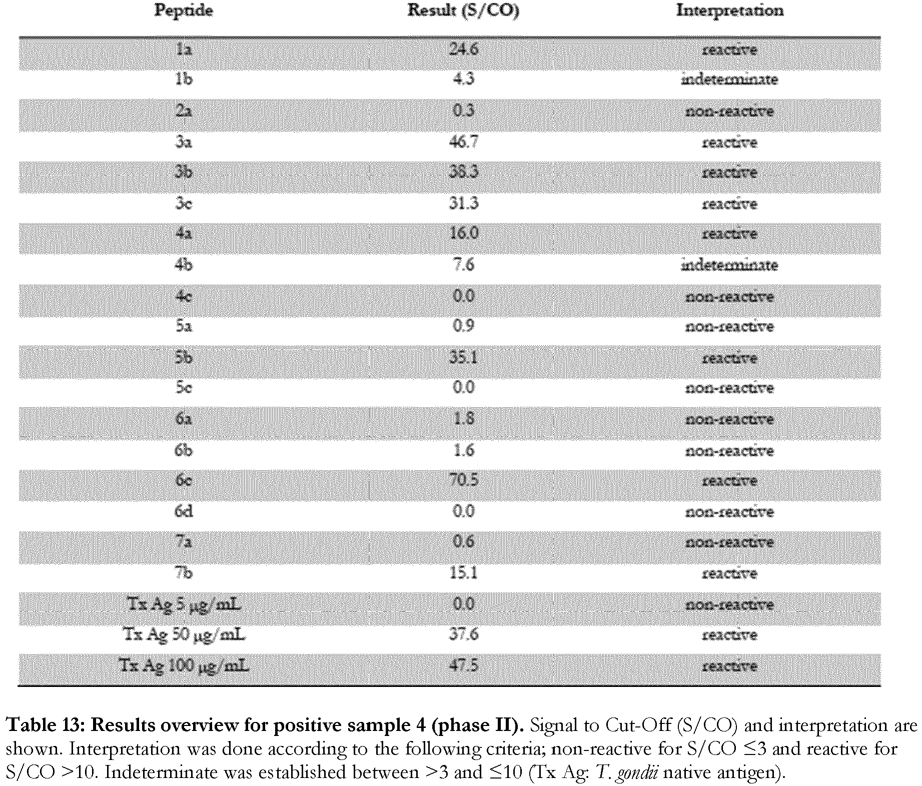

The objectives were, first, to ascertain if we could reproduce the results obtained with the multiplex ELISA during the collaboration with InfYnity Biomarkers. Then, if the first objective was accomplished, the second was to establish an in-house protocol to perform an indirect ELISA. The new indirect ELISA would use the synthetic peptides as antigens, thus validating the functionality of the selected peptides as new diagnostic tools for detecting *T. gondii* IgM-positive samples. Reproducing the multiplex parameters in singleplex ELISA.

During the collaboration with InfYnity Biomarkers, we showed that the selected peptides could be directly attached to the solid surface of the 96-well plates, instead of being used as a conjugate (which is the common approach used by other commercial available immunoassays, as seen in Table 8). Thus, we decided to design an indirect ELISA to validate the results obtained with the multiplex ELISA, both in phase I and II. The indirect approach allowed us to simplify the method, since we did not have to label all the peptides with peroxidase.

Several ELISA plates were coated with the five selected peptides at 100 µg/mL, following standard in-house coating procedures using single strip-detachable 96-well plates. The strip detachable 96-well plates allowed us to easily reassemble ELISA plates and customize the final ELISA plate to perform the functional assay (see Figure 7).

To validate the results obtained by multiplex ELISA, two positive and two negative samples that were tested in phase I were selected. Using the ELISA format shown in Figure 7, 1/50 samples and 1/1000 conjugate dilutions were evaluated. Once the ELISA protocol was completed, the optical density (OD) of each plate well was read using a 630 nm filter. Relative absorbance units were calculated by subtracting blank results (read at 450 nm with the blank well). The cut-off value was calculated as the average result of the absorbance of all negative samples (0.187 OD). Signal-to-noise ratio (SNR) was then calculated according to the absorbance value of each positive sample divided by the cut-off value. Results are shown in Figure 8.

The results of the singleplex ELISA showed a good correlation with those of the multiplex ELISA developed in collaboration with InfYnity Biomarkers. Peptides 3a, 3b, and 6c showed higher fluorescent units and SNR, followed by 3c and 7b. Because the units of the two techniques were not comparable, we calculated the standard deviation and extrapolated the result to a percentage value with respect to the highest value (data not shown). In doing so, we observed an interesting fac*T*. In multiplex ELISA, the standard deviation of sample #31 for 3a, 3b and 6c peptides was 979, which represented 14% with respect to the highest reactivity value (6912). The same determination in singleplex ELISA gave a standard deviation of 3.7, i.e. 28% with respect to the highest reactivity value (13.3). This difference (twice as high in the singleplex than in the multiplex) was not observed in sample 46; the difference could be attributed to sample degradation or to a manipulation error.

The multiplex ELISA had allowed us to establish the optimal working ranges for each assay condition studied (peptide, sample and conjugate concentrations) and to identify which were the best performing peptides. We next translated the assay conditions established with the multiplex ELISA to a singleplex forma*T*. However, due to the methodological differences between multiplex and singleplex, such as coating of the plates (peptide printing versus precipitation from carbonate buffer, among others), we decided to examine in more depth the assay conditions established by the multiplex ELISA and optimize them to a singleplex context with the objective of improving performance of the latter. To this end, we performed a checkerboard titration. In all immunoassays it is important to optimize the concentration of the sample and the antigens or the antibodies used to capture or detect the samples. If the sample or antigen/antibodies are too concentrated, there is a risk of saturation. On the contrary, if samples or antibodies are not concentrated enough, the signal will be weak and difficult to detec*T*. Checkerboard titration can be used to assess two variables at once: in this case, we decided to study the concentration of the peptide that would be used to coat the ELISA plates to capture the IgM anti-*T*. *gondii,* and the conjugate concentration. By running each well with a different ratio of peptide and conjugate, not only the optimal concentration of each one can be found, but also the optimal ratio of both concentrations.

An example of a checkerboard titration is shown in Figure 9. Each one of the columns 1-12 corresponds to different peptide dilution factors in decreasing order and rows A-H to conjugate dilution factors also in decreasing order. Table 14 summarizes the concentration range assessed for each peptide, the peptide concentration finally selected, the absorbance of the positive and negative samples that were used and the SNR. For all peptides, the best dilution of conjugate was 1/2000. Peptide 6c required the lowest peptide concentration, however it showed the highest SNR. In contrast, peptide 7b required the highest peptide concentration to reach a SNR of 1.9.

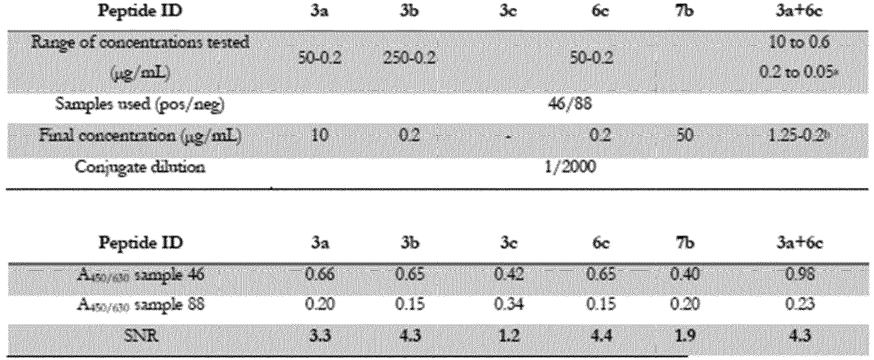

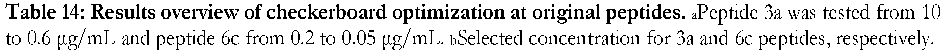

Once the optimal assay conditions of the indirect ELISA were established, the conditional panel (see Table 8) was evaluated with peptides 3a, 3b, 6c and 7b. Due to low volume, sample 49 was excluded from the study (n=93). Peptide 3c was excluded due to the low reactivity showed during the checkerboard (SNR of 1.2).

For the evaluation, three 96-well plates were coated with each peptide (3a, 3b, 6c, 7b) at the selected peptide concentrations. Each sample was evaluated in duplicate, including the blank. Optical density of each well was read using a 630 nm filter. Relative absorbance units were calculated by subtracting blank result (read at 450 nm). The net absorbance of each sample was reported, and its status (negative or positive) was used to generate a receiver operating characteristic curve (ROC curve) using Analyseit^{®} software. The ROC curve is a graphical plot that illustrates the diagnostic ability of a binary classifier system as its discrimination threshold is varied. Thus, the ROC curve was created by plotting the true positive proportion (TPP, also known as sensitivity) against the false positive proportion (FPP, also known as probability of false alarm or false positive, calculated as 1 - specificity, at various threshold settings).

Additionally, we wanted to evaluate whether the combination of two peptides could have synergy, thus increasing the SNR obtained individually. Peptide 7b was discarded as its reactivity to IgM positive samples was low. Peptide 6c was selected because it was the best candidate so far. To decide between peptides 3a and 3b, we set up a tentative cut-off (0.21) and analyzed the number of true positives, true negatives, false positives and false negatives obtained after screening with the conditional panel (data not shown). The number of false positives and false negatives results coincided, although peptide 3a showed two additional true positive results, and one additional true negative result. Therefore, we decided to test the combination of peptides 3a and 6c. A checkerboard was performed with a mixture of the two peptides at different concentrations. The checkerboard revealed that when both peptides were used together as antigens, a high SNR could be obtained with a concentration of 3a nearly 10 times lower than when 3a was used on its own (see Table 14). Once the assay conditions were established, the conditional panel (see Table 8) minus the sample 49 (n=93) was evaluated with the combination of peptides 3a and 6c. (see Figure 10).

As can be seen in Figure 10, peptide 7b as such was not a good candidate, because at some point the line approaches the non-discrimination area (random classification), which indicates that 7b does not reliably classify samples as positive or negative. Additionally, its area under the curve (AUC) is the lowest, 0.741 with a 95% confidence interval (CI) of 0.624 to 0.859 (see Table 15). Peptides 3a, 3b, and 6c showed better diagnostic performance, as their ROC curves are closer to the upper left corner (see Figure 10). That corner, also known as the 0.1 point or perfect classification, represents 100% sensitivity (no false negatives) and 100% specificity (no false positives). Additionally, their AUC values are around 0.85 or even higher in the case of peptide 6c and the combination of 3a and 6c which were both 0.89 with a 95% Cl of 0.82 to 0.95. (see Table 15).

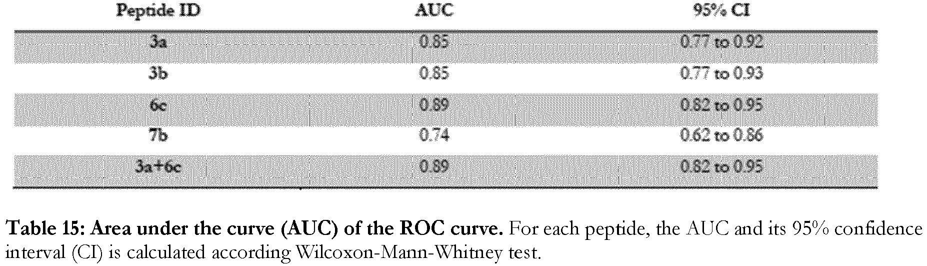

The ROC curve allowed us to establish the best threshold or cut-off level in which each peptide reaches its best diagnostic performance. Accuracy estimators, sensitivity (Se) and specificity (Sp) foreach peptide was calculated using Analyse-it^{®} software. We determined that the combination of peptides 3a and 6c acted synergistically in terms of sensitivity.

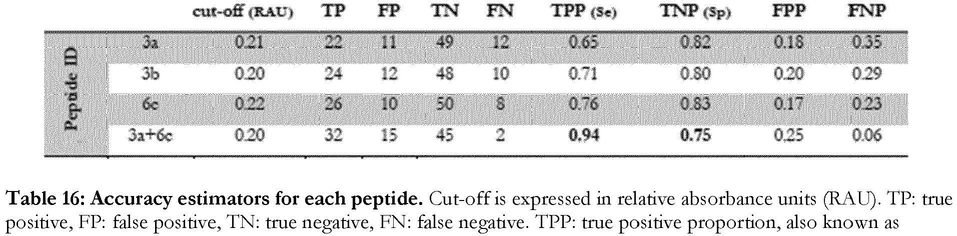

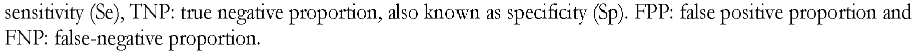

### Example 2. Peptide chimeras to enhance sensitivity

In the previous example, we showed that combining two peptides in solution enhanced reactivity compared to their individual use. In view of this, a logical next step was to design and evaluate chimeric peptides that combined the sequences of the better candidates identified in example 1. The rationale for designing the chimeric peptide was thus threefold: i) to increase IgM specificity, ii) to control peptide orientation on the solid surface (i.e., polystyrene plate or magnetic particles), and to assess if, using peptides reactive not only to IgM-positive but also to IgG-positive samples, one could increase the performance of the assay. Multimeric peptides, displaying several B and/or T cell epitopes on a single molecular scaffold, have shown to possess a wide array of biomedical applications as tools in drug design, targeted delivery, serodiagnosis, oncology and vaccinology. These chimeric molecules present demonstrable advantages, in that they are versatile, highly stable (i.e., compared to native proteins), easy to produce at moderate cost, and have good immunogenicity.

For that purpose, we first selected two best-performing sequences from the microarray study in example 1. Next, we produced synthetic versions of those peptides using SPPS. We also included peptide 7b, after having initially discarded it, because in the microarray study it showed specific reactivity to IgG-positive samples. Finally, we labelled with biotin some of the chimeric peptides to evaluate whether peptide orientation on the solid surface could affect the assay performance.

We used a singleplex ELISA to evaluate the performance of the new chimeric constructions with IgM anti-*T*. *gondii* positive samples. We selected the same approach as in example 1 to establish the optimal assay conditions for the new peptides.

### 2.1. Design and synthesis of chimeric peptides based on topper forming peptides 6c & 3a

Many immunoassays rely on the attachment of antigens onto solid surfaces such as polystyrene plates or magnetic particles to detect the molecule of interest. Short synthetic peptides that are easily produced through chemical synthesis are commonly used antigens because of the high specificity they confer. However, many of them show less than ideal ability to bind to solid surfaces. Multimeric peptide constructs, particularly those with dendrimeric (branched) arrangements, tend to be much better than linear ones in overcoming such limitations. This is probably because those peptides tend to adopt a more extended spatial organization than linear species, providing increased surface-binding properties and enhanced sensitivity, thus becoming more effective in disease diagnosis.

Multiepitope linear peptides contain more than one repeat of a given epitope in juxtaposed fashion. Multiepitope dendrimer peptides, for their part, have branched architectures with high molecular organization and stability. In dendrimers, the molecular scaffold is built around a core matrix to which different branches are tethered. Different amino acids are used for core formation, but lysine (Lys) is used preferentially because its two amino groups (α, Σ) can be utilized as branching points to generate multiplicity. In this work we have designed and produced both types of multiepitope peptides, i.e. constructs with various repeats of the same epitope (chimeric homotandems), in both linear and branched versions as well as constructs with more than one repeat of different epitopes, those always in linear fashion. Additionally, we produced peptide constructs to optimize peptide immobilization by biotin labelling. All peptides were synthesized in C-terminal carboxamide form using Fmoc solid phase synthesis, purified to >95% homogeneity using HPLC, and satisfactorily characterized using MS.

### 2.2. Chimeric homotandems

It is worth mentioning an interesting feature of peptide 6c, composed of a tandem repeat of eight residues (PPPNXQEL, wherein X can be any of aminoacids S or A). Given the presence of those block-tandem sequences in ROP1 protein we hypothesized that these eight residues might be involved in recognizing IgM. On that basis, we designed and produced other chimeric peptides with more than one repetition of the 6c sequence minus the two N-terminal residues (hereinafter named truncated 6c*) as a motif for three different constructions, namely i) homotandem with two consecutive stretches of truncated peptide 6c (ID 6c*2), ii) homotandem with two truncated 6c peptide sequences separated by a flexible spacer (8-amino-3,6-dioxaoctanoic acid (O2Oc)) (ID 6cO36c^{†}), and iii) branched bivalent peptide based on a lysine core from which two truncated 6c peptide sequences branched out (ID (6c*)2K3) (Figure 11).

### 2.3. Chimeric heterotandems

To assess whether the combination of peptides 3a and 6c in a single construction could enhance the reactivity achieved with the original sequences tested together in solution (see example 1), we designed and produced two chimeric peptides as follows i) a heterotandem construct combining truncated 6c and 3a sequences using 8-amino-3,6-dioxaoctanoic acid (O2Oc) as a flexible spacer between them (ID 6c3a), and ii) the reverse heterotandem version in which we merely changed the sequence order of truncated 6c and 3a peptides (ID 3a6c) (Figure 12). In table 17 we therein show each of the peptides indicated in sections 2.2, 2.3. and 2.4.

| | | | |
|---|---|---|---|
| 6c | EVPPPNAQELPPPNSQELPPPNSQELP | 2920 | |
| 6c* | PPPNAQELPPPNSQELPPPNSQELP | 2687 | |
| 3a | FLVAAALGGLAADQPENHQALAEPVTGVGEAGVSPVNEAG | 3828 | |
| 3b | AADQPENHQALAEPVTGVGEAGVSPVNEAGESYSSATSGVQ | 4010 | |
| 6c*₂ | PPPNAQELPPPNSQELPPPNSQELPPPNAQELPPPNSQELPPPNSQELP | 5261 | tandem (no spacer) |
| (6c)₂K₂ | PPPNAQELPPPNSQELPPPNSQELc-Mal KKK PPPNAQELPPPNSQELPPPNSQELc-Mal | 6091 | |
| 6cO₃6c^{†} | PPPNAQELPPNSQELPPPNSQELOOOPPPNAQELPPPNSQELPPPNSQELP | 5695 | tandem (spacer) |
| 6c3a | PPPNAQELPPPNSQELPPPNSQELOOOFLAVAAALGGLAADQPENHQALAEPVTGVGEAGVSPNEAG | 6836 | tandem heteromer |
| 3a6c | FLVAAALGGLAADQPENHQALAEPVTTGVGEAGVSPVNEAGOOOPPPNAQELPPPSQELPPPNQEL | 6836 | tandem heteromer |
| 3a biot | B-FLVAAALGGLAADQPENHQALAEPVTGVGEAGVSPVNEAG | 4052 | Biotin conjugated |
| 6c* biot | B-PPPNAQELPPNSQELPPPNSQELP | 2912 | Biotin conjugated |

| | | | |
|---|---|---|---|
| *minus residues EV at the N-terminus; only the PPPNXQEL repeat (X=A,S) ^{†}O=O₂Oc, 8-amino-3,6-dioxaoctanoic acid 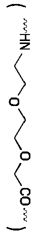 | | | |

### 2.4. Biotin-labeled peptides

In many instances, proteins or peptides are randomly immobilized by simple adhesion to immunoassay solid surfaces, which results in a loss of functional epitopes that are responsible for bestowing specificity for the molecule that we aim to capture. Biotin labelling has been used for several decades for protein and peptide orientation as well as for numerous laboratory research techniques (i.e., label, detect, and purify). The biotin moiety has a very strong affinity for streptavidin (Kd <10- 10M) and biotinylation is, therefore, an efficient method for specifically binding peptides to streptavidin-coated surfaces. Biotinylation can be performed either at the N- or C-terminus. At the N-terminus it can be conducted directly on the primary-terminal amino group, which was the strategy we selected.

We synthesized and purified different biotin-labelled peptides to study how distinct peptide orientations on the immunoassay surface could affect the reactivity of the resulting assay; i) biotinlabeled 3a sequence (ID 3ab), ii) biotin-labeled truncated 6c peptide (ID 6c*b), iii) 3a6c biotin-labeled heterotandem (ID 3a6c*b) and iv) biotin-labeled 7b peptide (ID 7bb).

### 2.5. Evaluation of chimeric peptides via enzyme-linked immunosorbent assay

Once we had synthesized the chimeric peptides, we evaluated their ability to detect IgMs compared with the original peptides. Initially, we established the optimal assay conditions. To do so, we reproduced the procedure used with the original sequences (see example 1). Since we knew the optimal assay conditions of the original sequences with which we designed the chimeric peptides, we established the different concentration ranges to be tested based on previous data. Thus, we used from 0.2 to 8 x 10-4 µg/mL for all chimeric peptides. For common IgM-IgG peptides, since they were being tested for the first time, we evaluated wider concentration ranges (from 50 to 0.2 µg/mL) (Table 18).

The checkerboard evaluation revealed that the signal-to-noise ratio (SNR) of chimeric peptides increased almost two-fold compared with that obtained by the combination of 3a and 6c peptides in solution (Table 18). The chimeric peptide that showed the lowest SNR ratio was (6c*2)K3. In contrast, 3a6c* showed the highest SNR. The common IgM-IgG sequences (peptides 8a and 9a) showed lower SNR compared with the original sequences tested in example 1, indicating they were not good candidates to be used as antigens.

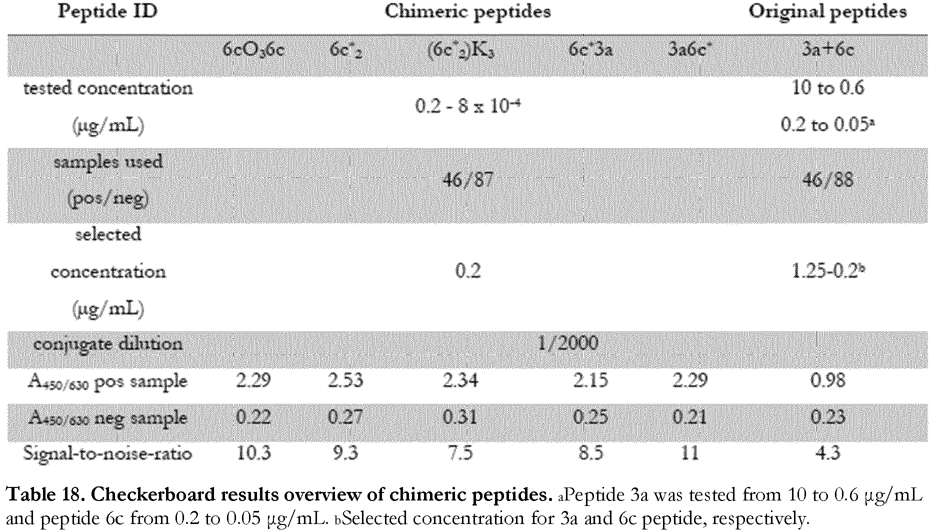

Once we evaluated the chimeric peptides with the checkerboard, we analysed their performance with the conditional sample panel (n=94).

The use of chimeric peptides did not increase assay performance compared with the peptide combination in solution (3a+6c), except for 3a6c (see Table 19) which reached the same number of true positives (TP), one true negative (TN), one false positive (FP) and one false native (FN) fewer than 3a+6c.

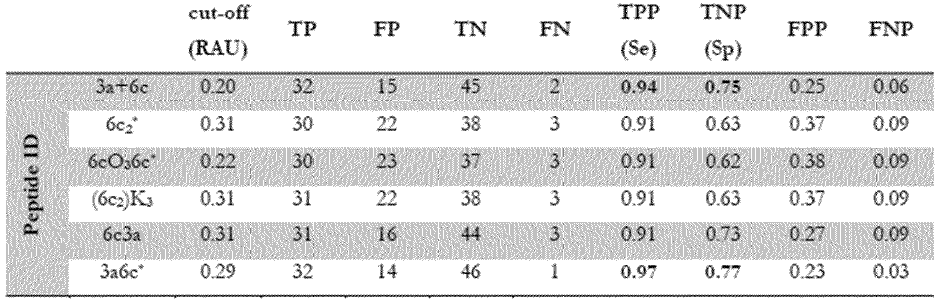

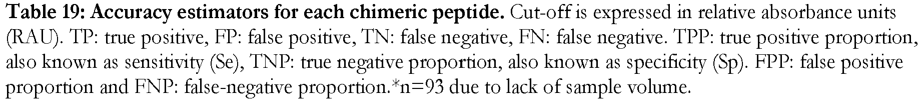

Figure 13 presents the ROC curve of all chimeric peptides. 6cO36c peptide runs at some point on the non-discrimination area, indicating that it is not able to classify samples according to their real status (sensitivity of 91% and specificity of 63%). Although the sensitivity is the same and the specificity is similar to that of 6c2, 6c3a, and (6c2)K3 peptides, none of the other ROC curves crossed the non-discriminatory line, which indicates that for this specific threshold, only 6cO36c peptide classified the samples incorrectly.

We also calculated the area under the curve (AUC) of each ROC with a 95% confidence interval (Table 20). Peptide 3a6c showed the highest AUC (0.90), followed by 6c3a peptide (0.88).

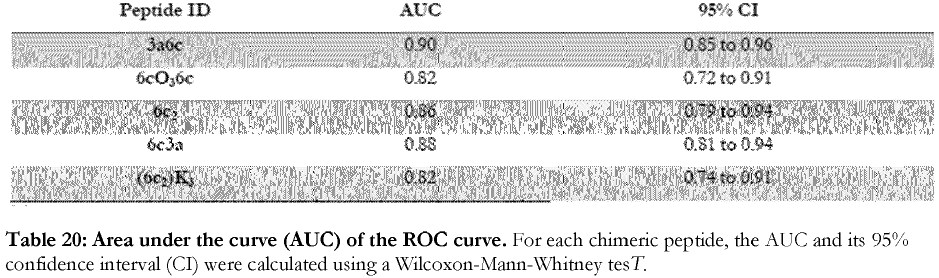

### 2.6. Evaluation of biotin-labelled peptides via ELISA

Until now, we have seen that 3a6c chimeric peptide, which combined the sequence of 3a and 6c peptides within the same molecule, increased assay performance compared with the performance obtained with the same peptides tested together in solution (3a+6c). As explained above, peptides that are randomly immobilized on the surface of ELISA plates are prone to lose by occlusion functional epitopes, with a direct impact on assay performance. Thus, we wanted to elucidate if we could obtain better assay performance by controlling orientation by using biotin-labelled peptides. Although we knew that the best candidate hitherto was the 3a6c chimeric peptide, we went on to evaluate the biotin-labelled truncated 6c peptide (6c*b). We performed a checkerboard to establish the optimal assay conditions.

Since we already knew the optimal conditions for its non-labelled analogue, we started with a peptide concentration of 2.5 µg/mL on a streptavidin-coated 96-well plate.

We performed this experiment using standard ELISA plates. When we did so, the absorbance of the blank was what we expected, and the absorbance of the strips that contained 2.5 µg/mL of 6c*b peptide, despite being lower than that obtained with 6c peptide was three times higher than the blank (data not shown).

## Claims

1. An antigen comprising an antigenic region of *Toxoplasma gondii,* wherein the antigenic region of *Toxoplasma gondii* comprises one or more amino acid sequences of SEQ ID: PPPNXQEL, wherein X can be any of amino acids S or A, and wherein the antigen is capable of specifically binding to *Toxoplasma gondii*-specific IgM antibodies.

2. The antigen of claim 1, wherein the antigenic region of *Toxoplasma gondii* comprises two or more amino acid sequences of SEQ ID: PPPNXQEL, wherein X can be any of amino acids S or A, and wherein the C-terminus of each amino acid sequence is linked to the N-terminal of the subsequent amino acid sequence through a simple covalent bond or they may employ a flexible linker domain, or polypeptide linkers consisting of small amino acids such as glycine, serine, threonine or alanine, at various lengths and combinations.

3. The antigen according to any one of claims 1 or 2, wherein the antigenic region comprises any one of the following sequences: SEQ ID NO 1 (antigen 6c), SEQ ID NO 2 (6c*), SEQ ID NO 3 (6c*2), and/or SEQ ID NO 4 (6cO36c), and wherein each of these sequences is capable of specifically binding to *Toxoplasma gondii*-specific IgM antibodies and preferably has a maximum length of 150 amino acids.

4. The antigen according to claim 1, wherein the antigen is a chimeric antigen comprising two or more different antigenic regions, one of these regions being a sequence selected from any one of claims 1 or 2, and the other antigenic region is selected from any one of SEQ ID NO 5 (3a) and/or SEQ ID NO 6 (3b), and wherein the C-terminus of the first antigenic region is linked to the N-terminal of the other antigenic region through a simple covalent bond or they may employ a flexible linker domain, or polypeptide linkers consisting of small amino acids such as glycine, serine, threonine or alanine, at various lengths and combinations.

5. The chimeric antigen of claim 4, comprising the amino acid sequence of SEQ ID NO: SEQ ID NO: 7 (3a6c) wherein this sequence is capable of specifically binding to *Toxoplasma gondii-*specific IgM antibodies and preferably has a maximum length of 150 amino acids.

6. The chimeric antigen according to claim 4, comprising the amino acid sequence of SEQ ID NO 8 (6c3a), wherein this sequence is capable of specifically binding to *Toxoplasma gondii*-specific IgM antibodies and preferably has a maximum length of 150 amino acids.

7. The chimeric antigen according to claim 4, wherein the antigen is selected from the list consisting of SEQ ID NO: 7 (3a6c) and/or SEQ ID NO 8 (6c3a).

8. *In vitro* use of any one of the antigens according to any one of claims 1 to 7, for for detecting anti-*T*. *gondii* IgMs in an isolated test sample, preferably a biological sample such as whole blood or part of whole blood, e.g., plasma or serum sample.

9. *In vitro* use of any one of the antigens according to any one of claims 1 to 7, for the diagnosis of acute toxoplasmosis in an isolated test or biological sample taken from a subject in need thereof.

10. Kits for the diagnosis of acute *Toxoplasma gondii* infection, comprising at least one antigen according to any one of claims 1 to 7.

11. Kits for detecting of *Toxoplasma gondii*-specific IgM antibodies in an isolated test or biological sample, containing at least one antigen according to any claim from 1 to 7.

12. Antigens according to any claim from 1 to 7 for use in therapy.

13. An immunoassay device or kit that employs an antigen to detect IgM antibodies from *Toxoplasma gondii* infection, wherein the immunoassay format contains at least one antigen according to any claim from 1 to 7.

14. The immunoassay device or kit of claim 13, wherein the antigens are labelled, or the device further comprises labelled antibodies; wherein the labels are selected from enzymatic, fluorescent, chemiluminescent, radioactive, or dye molecules.

15. The immunoassay device or kit of any of claims 13 or 14, wherein the device comprises a solid support to which the antigen/s is bound.

16. The kit according to claim 10 or 11 or the immunoassay according to any of claims 13 to 15, wherein the kit contains in separate containers a combination of antigens (either already bound to a solid matrix or separate with reagents for binding them to the matrix), control antibody formulations (positive and/or negative), labelled antibodies when the assay format requires same and signal generating reagents (e.g., enzyme substrate) if the label does not generate a signal directly and optionally instructions for carrying out the assay usually will be included in the kit.
